# EUROPEAN PATENT SPECIFICATION

(11) **EP 2 676 664 B1**
(45) Date of publication and mention of the grant of the patent: **21.06.2017**
(21) Application number: 12747713.1
(22) Date of filing: 17.02.2012
(51) Int. Cl.: A61K 31/198, A61K 31/513, A61P 35/00

(54) **POTENTIATOR OF ANTITUMOR ACTIVITY OF CHEMOTHERAPEUTIC AGENT**
VERSTÄRKER DER ANTITUMORWIRKUNG EINES CHEMOTHERAPEUTISCHEN MITTELS
POTENTIALISATEUR DE L'ACTIVITÉ ANTITUMORALE D'UN AGENT CHIMIOTHÉRAPIQUE

(30) Priority: 17.02.2011 JP 2011032511; 29.09.2011 JP 2011215605
(43) Date of publication of application: 25.12.2013
(73) Proprietor: EA Pharma Co., Ltd., Tokyo 104-0042 (JP); KURUME UNIVERSITY, Kurume-shi Fukuoka 830-0011 (JP)
(72) Inventor: NISHITANI, Shinobu, Kawasaki-shi Kanagawa 210-8681 (JP); YANO, Hirohisa, Kurume-shi Fukuoka 830-0011 (JP)
(74) Representative: Nicholls, Kathryn Margaret
(86) International application number: PCT/JP2012/053757
(87) International publication number: WO 2012/111790

(56) References cited:
- WO-A1-03/045372
- WO-A2-2009/132320
- JP-A- 3 068 514
- JP-A- H11 508 282
- JP-A- 2004 339 151
- JP-A- 2010 083 858

## Description

### Technical Field

The present invention relates to an agent for potentiating an antitumor activity of a chemotherapeutic agent for cancer containing cancer stem cells, which contains a branched chain amino acid, an agent for inducing differentiation of cancer stem cells, which contains a branched chain amino acid, which contains a branched chain amino acid, and an agent for suppressing or treating metastasis or recurrence of cancer containing cancer stem cells, which contains a branched chain amino acid, and a chemotherapeutic agent in combination, and the like.

### Background Art

Cancer stem cell is one of the cancer cell subpopulations contained in tumors, has self-replication competence and multipotency, and has cancer-evoking competence when transferred orthotopically (non-patent document 1).

In various cancers, the presence of markers expressed specifically in cancer stem cells is known, and one can evaluate whether or not the cancer cell is a cancer stem cell based on the expression of such markers. As markers of hepatic cancer stem cells, EpCAM, AFP and the like are known (non-patent document 2). As markers of colorectal cancer stem cells, CD44, CD24 and the like are known (non-patent document 3). As markers of breast cancer stem cells, CD44, CD24, EpCAM and the like are known (non-patent document 1).

Since cancer stem cells are generally slow in growth speed, they show resistance to chemotherapeutic agents. On the contrary, since differentiated cancer cells (cancer non-stem cells) have a fast growth speed, they are highly sensitive to chemotherapeutic agents. Since many of the chemotherapeutic agents target the growth mechanism of cancer cells, a treatment by a chemotherapeutic agent targeting only the cancer non-stem cells may not be able to kill cancer stem cells effectively. Even when most of the cancer cells show regression by treatments with chemotherapeutic agents, an extremely small number of potentially remaining cancer stem cells can cause recurrence, which is considered the cause of metastasis or recurrence that occurs often in cancer. It is therefore expected that promotion of the differentiation of cancer stem cells into cancer non-stem cells and enhancement of the sensitivity to chemotherapeutic agents will enable efficient treatment or prevention of the whole cancer including cancer stem cells.

Branched chain amino acids (BCAAs) have heretofore been reported to suppress development and progression of hepatic cancer (patent documents 1 - 4). It has further been reported that a long-term administration of the branched chain amino acid suppresses recurrence of hepatoma (non-patent document 4). Moreover, it has been reported that the branched chain amino acid suppresses activation of Akt, which in turn suppresses development and progression of cancer in patients with hyperinsulinemia or with a risk of hyperinsulinemia (patent document 5). It has further been reported that the branched chain amino acid potentiates anti-hepatitis C virus activity of IFN agents (patent document 6).

Nevertheless, the effect of the branched chain amino acid on the differentiation of cancer stem cells into cancer non-stem cells has not been clarified to date.

### Document List

### Patent Documents

patent document 1: WO2003/16208
patent document 2: WO2004/058243
patent document 3: WO2007/018278
patent document 4: WO2006/06729
patent document 5: WO2007/018281
patent document 6: WO2007/013677

### Non-patent Documents

non-patent document 1: PNAS, vol. 100, No. 7, pages 3983-3988, 2003
non-patent document 2: Cancer Research, vol. 65, No. 8, pages 1451-1461, 2008
non-patent document 3: PNAS, vol. 171, No. 8, pages 3722-3727, 2010
non-patent document 4: Nisshoshi (Journal of Japanese Society of Gastroenterology), vol. 105, pages 808-816, 2008

### SUMMARY OF THE INVENTION

### Problems to be Solved by the Invention

The present invention aims to develop a technique for promoting differentiation of cancer stem cells into cancer non-stem cells and enhancing sensitivity to chemotherapeutic agents, and provide a means for effectively treating cancer containing cancer stem cells and preventing cancer recurrence based on said technique.

### Means of Solving the Problems

The present inventors have conducted intensive studies in an attempt to solve the aforementioned problems and found that particular BCAA promotes differentiation of cancer stem cells into cancer non-stem cells and enhances sensitivity to chemotherapeutic agents, which resulted in the completion of the present invention.

Accordingly, the present invention provides the following.
[1] An agent for potentiating an antitumor activity of a chemotherapeutic agent against a cancer containing cancer stem cells, which comprises at least one kind of branched chain amino acid selected from isoleucine, leucine and valine, or a salt thereof.
[2] The agent of [1], which is administered in combination with the chemotherapeutic agent to a mammal affected with a cancer containing cancer stem cells, or a mammal with a history of having a cancer containing cancer stem cells.
[3] The agent of [1], comprising isoleucine or a salt thereof, leucine or a salt thereof, and valine or a salt thereof.
[4] The agent of [1], wherein the chemotherapeutic agent is an antimetabolite, an alkylating agent, an antitumor antibiotic, a plant alkaloid, or a molecular target therapeutic agent.
[5] The agent of [1], wherein the chemotherapeutic agent is 5FU.
[6] The agent of [1], wherein the cancer containing cancer stem cells is a chemotherapeutic agent-resistant cancer.
[7] The agent of [1], wherein the cancer containing cancer stem cells is gastric cancer, hepatic cancer, breast cancer or colorectal cancer.
[8] An agent for treating a cancer containing cancer stem cells, which comprises a combination of a chemotherapeutic agent and at least one kind of branched chain amino acid selected from isoleucine, leucine and valine, or a salt thereof.
[9] The agent of [8], comprising isoleucine or a salt thereof, leucine or a salt thereof, and valine or a salt thereof.
[10] The agent of [8], wherein the chemotherapeutic agent is an antimetabolite, an alkylating agent, an antitumor antibiotic, a plant alkaloid, or a molecular target therapeutic agent.
[11] The agent of [8], wherein the chemotherapeutic agent is 5FU.
[12] The agent of [8], wherein the cancer containing cancer stem cells is a chemotherapeutic agent-resistant cancer.
[13] The agent of [8], wherein the cancer containing cancer stem cells is gastric cancer, hepatic cancer, breast cancer or colorectal cancer.
[14] An agent for preventing metastasis or recurrence of a cancer containing cancer stem cells, which comprises a combination of a chemotherapeutic agent and at least one kind of branched chain amino acid selected from isoleucine, leucine and valine, or a salt thereof.
[15] The agent of [14], comprising isoleucine or a salt thereof, leucine or a salt thereof, and valine or a salt thereof.
[16] The agent of [14], wherein the chemotherapeutic agent is an antimetabolite, an alkylating agent, an antitumor antibiotic, a plant alkaloid, or a molecular target therapeutic agent.
[17] The agent of [14], wherein the chemotherapeutic agent is 5FU.
[18] The agent of [14], wherein the cancer containing cancer stem cells is a chemotherapeutic agent-resistant cancer.
[19] The agent of [14], wherein the cancer containing cancer stem cells is gastric cancer, hepatic cancer, breast cancer or colorectal cancer.
[20] An agent for inducing differentiation of a cancer stem cell, which comprises at least one kind of branched chain amino acid selected from isoleucine, leucine and valine, or a salt thereof.
[21] The agent of [20], comprising isoleucine or a salt thereof, leucine or a salt thereof, and valine or a salt thereof.
[22] The agent of [20], wherein the cancer stem cell is a gastric cancer stem cell, a hepatic cancer stem cell or a colorectal cancer stem cell.
[23] An agent for suppressing metastasis of a cancer containing cancer stem cells, which comprises at least one kind of branched chain amino acid selected from isoleucine, leucine and valine, or a salt thereof.
[24] The agent of [23], comprising isoleucine or a salt thereof, leucine or a salt thereof, and valine or a salt thereof.
[25] The agent of [23], wherein the cancer containing cancer stem cells is gastric cancer, hepatic cancer, breast cancer or colorectal cancer.
[26] At least one kind of branched chain amino acid selected from isoleucine, leucine and valine, or a salt thereof, for use in potentiating an antitumor activity of a chemotherapeutic agent.
[27] A combination for use in treating a cancer containing cancer stem cells, which comprises at least one kind of branched chain amino acid selected from isoleucine, leucine and valine, or a salt thereof, and a chemotherapeutic agent.
[28] A combination for use in preventing metastasis or recurrence of a cancer containing cancer stem cells, which comprises at least one kind of branched chain amino acid selected from isoleucine, leucine and valine, or a salt thereof, and a chemotherapeutic agent.
[29] At least one kind of branched chain amino acid selected from isoleucine, leucine and valine, or a salt thereof, for use in inducing differentiation of a cancer stem cell.
[30] At least one kind of branched chain amino acid selected from isoleucine, leucine and valine, or a salt thereof, for use in suppressing metastasis of a cancer containing cancer stem cells.
[31] A method of potentiating an antitumor activity of a chemotherapeutic agent in a mammal, comprising administering an effective amount of at least one kind of branched chain amino acid selected from isoleucine, leucine and valine, or a salt thereof, to said mammal.
[32] A method of treating a cancer containing cancer stem cells in a mammal, comprising administering effective amounts of at least one kind of branched chain amino acid selected from isoleucine, leucine and valine, or a salt thereof, and a chemotherapeutic agent to said mammal.
[33] A method of preventing metastasis or recurrence of a cancer containing cancer stem cells in a mammal, comprising administering effective amounts of at least one kind of branched chain amino acid selected from isoleucine, leucine and valine, or a salt thereof, and a chemotherapeutic agent to said mammal.
[34] A method of inducing differentiation of a cancer stem cell in a mammal, comprising administering an effective amount of at least one kind of branched chain amino acid selected from isoleucine, leucine and valine, or a salt thereof, to said mammal.
[35] A method of suppressing metastasis of a cancer containing cancer stem cells in a mammal, comprising administering an effective amount of at least one kind of branched chain amino acid selected from isoleucine, leucine and valine, or a salt thereof, to said mammal.

### Effect of the Invention

Since branched chain amino acid promotes differentiation of cancer stem cells into cancer non-stem cells and enhances sensitivity to chemotherapeutic agents, cancer can be effectively treated by administering the branched chain amino acid and a chemotherapeutic agent in combination to a patient affected with a cancer containing cancer stem cells.

In addition, metastasis and recurrence of cancer can be effectively prevented by administering branched chain amino acid in combination with a chemotherapeutic agent to a patient with a history of having a cancer containing cancer stem cells.

### Brief Description of the Drawings

Fig. 1 shows the effect of BCAA on mRNA expression of EpCAM1, AFP and CYP3A4 in HAK4 cells, wherein the vertical axis shows a relative mRNA expression level of each gene, provided that expression upon LC2.5% treatment is 1. T-test, n=6, *p<0.05, mean+SE.
Fig. 2 shows induction of apoptosis of HAK4 cells by a combined use of 5FU and BCAA, wherein the vertical axis shows the percentage of Annexin V positive cells. T-test, n=7, *p<0.05, mean+SE.
Fig. 3 shows the effect of BCAA on mRNA expression of EpCAM1, AFP and CYP3A4 in HAK1B cells, wherein the vertical axis shows a relative mRNA expression level of each gene, provided that expression upon LC2.5% treatment is 1. T-test, n=6, *p<0.05, mean+SE.
Fig. 4 shows time-course changes in the tumor volume of Balb/c nude mouse transplanted with HAK1B cells, wherein group 1: vehicle + casein-mixed feed (control group), group 2: 5FU + casein-mixed feed, group 3: vehicle + BCAA-mixed feed, group 4: 5FU + BCAA-mixed feed.
Fig. 5 shows the tumor volume on day 15. Dunnet test, n=6, *p<0.05, ***p<0.001, mean+SE.
Fig. 6 shows the tumor volume on day 15. Dunnet test, n=6, *p<0.05, ***p<0.001, mean+SE.
Fig. 7 shows the effect of BCAA on CD44 expression in HCT116 cells, wherein the vertical axis shows the percentage of CD44 positive cells. T-test, n=7, *p<0.05, mean+SE.
Fig. 8 shows induction of apoptosis of HCT116 cells by a combined use of 5FU and BCAA, wherein the vertical axis shows the percentage of Annexin V positive cells. T-test, n=14, *p<0.05, mean+SE.
Fig. 9 shows the effect of BCAA on liver metastasis of HCT116 cells, wherein the vertical axis shows the number of liver metastasis per one mouse.
Fig. 10 shows induction of cell death of MKN45 cells by a combined use of 5FU and BCAA, wherein the vertical axis shows the relative value of OD450 nm. T-test, *p<0.05, n=8.
Fig. 11 shows induction of apoptosis of MKN45 cells by a combined use of 5FU and BCAA, wherein the vertical axis shows the percentage of apoptotic cells. T-test, *p<0.05, n=8.
Fig. 12 shows the effect of BCAA on CD44 expression in MKN45 cells, wherein the vertical axis shows the percentage of CD44 positive cells. T-test, *p<0.01, n=8.
Fig. 13 shows tumor volume on 6 weeks later. Dunnet test, n=6, **p<0.01, mean+SE.
Fig. 14 shows suppression of the growth of MDA-MB231 cells by a combined use of 5FU and BCAA. Student test vs 5FU, ***p<0.001, mean+SE.
Fig. 15 shows the effect of BCAA on CD44 expression in MDA-MB231 cells, wherein the vertical axis shows the percentage of CD44 positive cells. Student test vs RPMI-10%, mean+SE.
Fig. 16 shows the effect of BCAA on EpCAM mRNA expression in HAK1B cells in vivo. T-test, n=6, *p<0.05.
Fig. 17 shows the effect of BCAA on mRNA expression of CD44, nanog, and ABCB5 in MKN45 cells in vivo. Dunnet test, n=8, *p<0.05, **p<0.01, *** p<0.005 v.s. control.

### Description of Embodiments

The present invention provides an agent (or composition) for potentiating an antitumor activity of a chemotherapeutic agent against a cancer containing the cancer stem cells and/or an agent (or composition) for inducing differentiation of cancer stem cells, which comprises at least one kind of branched chain amino acid selected from isoleucine, leucine and valine, or a salt thereof (preferably isoleucine or a salt thereof, leucine or a salt thereof, and valine or a salt thereof) (hereinafter these preparations are also to be referred to as the agent or composition of the present invention).

In a further embodiment, the agent (or composition) of the present invention can be an agent (or composition) for suppressing metastasis of a cancer containing cancer stem cells.

The agent or composition of the present invention has been completed based on the finding that at least one kind of branched chain amino acid selected from isoleucine, leucine and valine, or a salt thereof (preferably a combination comprising isoleucine or a salt thereof, leucine or a salt thereof, and valine or a salt thereof) promotes differentiation of cancer stem cells into cancer non-stem cells, and, by this effect, potentiates an antitumor activity of a chemotherapeutic agent against a cancer containing cancer stem cells. Since cancer stem cells are generally slow in the growth speed, they show resistance to chemotherapeutic agents. On the contrary, since differentiated cancer cells (cancer non-stem cells) generally have a fast growth speed, they are highly sensitive to chemotherapeutic agents. Therefore, application of at least one kind of branched chain amino acid selected from isoleucine, leucine and valine, or a salt thereof (preferably a combination comprising isoleucine or a salt thereof, leucine or a salt thereof, and valine or a salt thereof) to a cancer containing cancer stem cells promotes differentiation of the cancer stem cells into cancer non-stem cells, which in turn enhances sensitivity of the cancer to chemotherapeutic agents and eventually enhances an antitumor activity of chemotherapeutic agents against said cancer.

In a further embodiment, the agent or composition of the present invention has been completed based on the finding that at least one kind of branched chain amino acid selected from isoleucine, leucine and valine, or a salt thereof (preferably a combination comprising isoleucine or a salt thereof, leucine or a salt thereof, and valine or a salt thereof) promotes differentiation of cancer stem cells into cancer non-stem cells, and, by this effect, suppresses metastatic ability of a cancer containing cancer stem cells.

In the present invention, the cancer stem cell means a cancer cell having self-replication competence and multipotency, and having tumor-evoking competence when transferred orthotopically (the tumor formed here is a phenocopy of a tumor of the origin from which the cancer stem cell is derived) (Nat Rev Cancer, vol. 5, pages 425-436, 2006).

In various cancers, since the presence of markers expressed specifically for cancer stem cells is known, one can evaluate whether or not the cancer cell is a cancer stem cell or whether a cancer contains cancer stem cells based on the expression of such markers. In one embodiment, hepatic cancer stem cell is EpCAM⁺AFP⁺ (Cancer Research, vol. 65, No. 8, pages 1451-1461, 2008). In one embodiment, colorectal cancer stem cell is CD44⁺CD24⁺ (PNAS, vol. 171, No. 8, pages 3722-3727, 2010). In one embodiment, breast cancer stem cell is CD44⁺CD24^{low+}EpCAM⁺ (PNAS, vol. 100, No. 7, pages 3982-3988, 2003). In one embodiment, pancreatic cancer stem cell is CD44⁺. Here, the expression of these markers is evaluated by flow cytometry or immunohistochemical staining using specific antibodies to these marker proteins, and is determined to be the marker expression positive (+) when specific staining is found by at least either one method. Since specific antibodies to these marker proteins, which are useful for flow cytometry or immunohistochemical staining are widely commercially available, those of ordinary skill in the art can easily evaluate whether or not cancer cell is a cancer stem cell, or whether cancer contains a cancer stem cell by using such antibodies.

In a further embodiment, gastric cancer stem cell is CD44⁺.

Moreover, differentiation of cancer stem cells into cancer non-stem cells can be evaluated using, as an index, a decrease in the expression of the markers of cancer stem cells. When expression of the markers of cancer stem cells in a cancer decreases after a treatment under particular conditions as compared to that before the treatment, it can be considered that differentiation of cancer stem cells into cancer non-stem cells was induced by the treatment. When either the ratio of the number of cells expressing the marker protein of cancer stem cells relative to the total cancer cells or the amount of the marker protein of cancer stem cells relative to the cancer as a whole decreases, the expression of the marker of cancer stem cells is determined to have decreased.

In the present invention, cancer encompasses cancers derived from any tissues. Examples of cancer include, but are not limited to, hepatic cancer, colorectal cancer, renal cancer, melanoma, pancreatic cancer, thyroid cancer, gastric cancer, lung cancer (small cell lung cancer, non-small cell lung cancer), brain tumor, uterine cancer, breast cancer, multiple osteosarcoma, ovarian cancer, chronic leukemia, prostate cancer, acute lymphoblastic leukemia, germinoma, acute myeloid leukemia, malignant lymphoma, choriocarcinoma, pediatric malignancy, gall bladder or·bile duct cancer and the like.

In one embodiment, the cancer containing cancer stem cells, to which the agent of the present invention is applied, is resistant to a chemotherapeutic agent. Being "resistant to a chemotherapeutic agent" means the property of not showing a significantly detectable response when a chemotherapeutic agent is administered at the maximum dose admitted for application to human. As mentioned above, a chemotherapeutic agent-resistant cancer may contain many cancer stem cells, since cancer stem cells are slow in the growth speed and resistance to chemotherapeutic agents. Therefore, it is expected that application of the agent or composition of the present invention to a cancer resistant to a particular chemotherapeutic agent promotes differentiation of cancer stem cells into cancer non-stem cells, and increases the sensitivity of the cancer to said chemotherapeutic agent.

Among the aforementioned cancers, hepatic cancer, colorectal cancer, renal cancer, melanoma, pancreatic cancer, thyroid cancer, gastric cancer, lung cancer, breast cancer and non-small cell lung cancer are known to generally show low sensitivity to chemotherapeutic agents, and react poorly to the chemotherapeutic agents. Therefore, it is expected that application of the agent or composition of the present invention to these cancers (preferably gastric cancer, hepatic cancer, breast cancer or colorectal cancer) to promote differentiation of cancer stem cells contained in these cancers increases the sensitivity of these cancers to chemotherapeutic agents.

In the present invention, the chemotherapeutic agent refers to a compound that directly acts on cancer cells, and has an ability to kill them by suppressing division thereof. Examples of the chemotherapeutic agent include antimetabolites, platinum preparations, alkylating agents, antitumor antibiotics, plant alkaloids, molecular target therapeutic agents and the like.

The antimetabolite refers to a chemotherapeutic agent having a chemical structure similar to that of a substance to be a material of nucleic acid when cancer cells are divided and proliferate, which prevents synthesis of DNA, inhibits metabolism of cancer cells and suppresses proliferation. Examples of the antimetabolite include pyrimidine antimetabolites such as 5-fluorouracil (5FU), tegafur, carmofur, doxifluridine, broxuridine, cytarabine, enocitabine, hydroxycarbamide, methotrexate, fludarabine phosphate and the like; purine antimetabolites such as 6-mercaptopurine, 6-thioguanine, thioinosine, gemcitabine hydrochloride etc., and the like. In one embodiment, the antimetabolite is preferably a pyrimidine antimetabolite, more preferably 5-fluorouracil or tegafur.

The platinum preparation refers to a chemotherapeutic agent which exhibits an effect of suppressing proliferation of cancer cells by binding to the N-7-position of guanine and adenine, which are DNA constituting bases, via two chlorine atom moieties, forming crosslinks in the DNA chain, and inhibiting DNA synthesis. Examples of the platinum preparation include cisplatin, carboplatin, nedaplatin, oxaliplatin and the like.

The alkylating agent refers to a chemotherapeutic agent which exhibits an effect of suppressing proliferation of cancer cells by cleaving DNA by alkylation. Examples of the alkylating agent include nitrogen mustard alkylating agents such as nitrogen mustard, nitrogen mustard N-oxide, chlorambucil and the like; ethylenimine derivatives such as carboquone, thiotepa and the like; sulfonates such as busulfan, improsulfan tosylate and the like; nitrosourea derivatives such as nimustine hydrochloride etc., and the like.

Examples of the antitumor antibiotic include anthracycline antibiotic antitumor agents such as mitomycin C, bleomycin, peplomycin, daunorubicin, aclarubicin, doxorubicin, pirarubicin, THP-adriamycin, 4'-epidoxorubicin, epirubicin and the like; chromomycin A₃, actinomycin D and the like.

Examples of the plant alkaloid include vinca alkaloids such as vinblastine, vincristine, vindesine and the like; epipodophyllotoxins such as etoposide, teniposide and the like; taxane alkaloids such as paclitaxel, docetaxel etc., and the like.

The molecule target therapeutic agent refers to a compound having an activity to kill cancer cells by targeting and inhibiting a molecule involved in the proliferation of cancer cells. Examples of the molecule target therapeutic agent include imatinib, gefitinib, erlotinib, vandetanib, sunitinib, sorafenib, rituximab, cetuximab, infliximab, trastuzumab, bevacizumab and the like.

In one embodiment, the agent or composition of the present invention is for potentiating the antitumor activity of an antimetabolite (preferably pyrimidine antimetabolite, more preferably 5-fluorouracil or tegafur) against hepatic cancer containing hepatic cancer stem cells.

In one embodiment, the agent or composition of the present invention is for potentiating the antitumor activity of an antimetabolite (preferably pyrimidine antimetabolite, more preferably 5-fluorouracil or tegafur) against colorectal cancer containing colorectal cancer stem cells.

In one embodiment, the agent or composition of the present invention is for potentiating the antitumor activity of an antimetabolite (preferably pyrimidine antimetabolite, more preferably 5-fluorouracil or tegafur) against gastric cancer containing gastric cancer stem cells, colon cancer containing colon cancer stem cells, cervical cancer containing cervical cancer stem cells, endometrial cancer containing endometrial cancer stem cells, gastrointestinal cancer containing gastrointestinal cancer stem cells, pancreatic cancer containing pancreatic cancer stem cells, rectal cancer containing rectal cancer stem cells, breast cancer containing breast cancer stem cells, or ovarian cancer containing ovarian cancer stem cells.

Isoleucine, leucine and valine, which are the active ingredients (branched-chain amino acid) of the agent or composition of the present invention, may be any of an L-form, a D-form and a DL-form, preferably an L-form or a DL-form, more preferably an L-form.

Isoleucine, leucine and valine can be used not only in a free form but also as a salt. Examples of the salt form include acid addition salt, base addition salt and the like, and any form can be taken as long as it is a chemically acceptable salt. Since the agent or composition of the present invention is generally used for medical purposes, the salt form is preferably a pharmaceutically acceptable salt.

Examples of the pharmaceutically acceptable salt include a salt with an acid and a salt with a base. Examples of the acid that is added to isoleucine, leucine or valine to form a salt acceptable as a pharmaceutical product include inorganic acids such as hydrogen chloride, hydrogen bromide, sulfuric acid, phosphoric acid and the like, organic acids such as acetic acid, lactic acid, citric acid, tartaric acid, maleic acid, fumaric acid or monomethylsulfuric acid and the like. Examples of the base which is added to isoleucine, leucine or valine to form a salt acceptable as a pharmaceutical include hydroxide of metal such as sodium, potassium and the like, carbonate of metal such as calcium and the like, an inorganic base such as ammonia and the like, and an organic base such as ethylenediamine, propylenediamine, ethanolamine, monoalkylethanolamine, dialkylethanolamine, diethanolamine, triethanolamine and the like.

The agent or composition of the present invention only needs to contain at least one kind of branched chain amino acids selected from isoleucine, leucine and valine, or a salt thereof, and the following embodiments are encompassed in the present invention:
an embodiment wherein only isoleucine or a salt thereof is contained as a branched chain amino acid;
an embodiment wherein only leucine or a salt thereof is contained as a branched chain amino acid;
an embodiment wherein only valine or a salt thereof is contained as a branched chain amino acid;
an embodiment wherein isoleucine or a salt thereof and leucine or a salt thereof are contained as branched chain amino acids; an embodiment wherein isoleucine or a salt thereof and valine or a salt thereof are contained as branched chain amino acids; an embodiment wherein leucine or a salt thereof and valine or a salt thereof are contained as branched chain amino acids; and an embodiment wherein isoleucine or a salt thereof, leucine or a salt thereof, and valine or a salt thereof are contained as branched chain amino acids.

The agent or composition of the present invention preferably contains isoleucine or a salt thereof, leucine or a salt thereof, and valine or a salt thereof.

The mixing ratio (weight ratio) of the branched chain amino acid contained in the agent or composition of the present invention can be appropriately adjusted by those of ordinary skill in the art, within the range where the agent of the present invention has a desired activity (e.g., activity to induce differentiation of cancer stem cells, or activity to potentiate the antitumor activity of a chemotherapeutic agent against a cancer containing cancer stem cells). For example, in the embodiment where isoleucine or a salt thereof, leucine or a salt thereof, and valine or a salt thereof are contained, the mixing ratio of the three kinds of amino acids (isoleucine:leucine:valine) in a weight ratio is generally 1:1 - 3:0.5 - 2.0, preferably 1:1.5 - 2.5:0.8 - 1.5, more preferably 1:1.9 - 2.2:1.1 - 1.3, most preferably 1:2:1.2. When the agent of the present invention contains a salt of isoleucine, a salt of leucine or a salt of valine, the weight ratio is calculated by converting the salt of each branched chain amino acid to a free form.

The agent and composition of the present invention can be administered to an animal (preferably mammal such as human and the like).

When the agent or composition of the present invention is used as an agent (or composition) for potentiating the antitumor activity of a chemotherapeutic agent against cancer containing cancer stem cells, or an agent (or composition) for inducing differentiation of cancer stem cells, the mode of administration and dosage form thereof may be oral administration or parenteral administration, and as an agent for oral administration, solids such as powder, granule, capsule, tablet, chewable agent and the like and liquids such as solution, syrup and the like can be mentioned, and as an agent for parenteral administration, injection, transfusion, transnasal or transpulmonary spray and the like can be mentioned. The agent or composition of the present invention can be formulated as a medicament in these dosage forms by a conventional method.

From the aspect of reducing a burden on patients, the branched chain amino acid is preferably administered orally to the subject. When oral administration to the patients is difficult, the branched chain amino acid can be intravenously or transarterially administered as an amino acid infusion.

When necessary for formulation, the agent of the present invention is produced by mixing with appropriate pharmacologically acceptable carriers, such as excipient, binder, lubricant, solvent, disintegrant, dissolution aids, suspending agent, emulsifier, isotonizing agent, stabilizer, soothing agent, preservative, antioxidant, corrigent, coloring agent and the like. Furthermore, the composition of the present invention can be prepared together with the above-mentioned carrier.

As the excipient, organic excipients such as saccharides (e.g. lactose, glucose, D-mannitol etc.), starches, celluloses (e.g. crystalline cellulose etc.), and the like, inorganic excipients such as calcium carbonate, kaolin and the like, and the like can be mentioned; as the binder, gelatinized starch, gelatin, gum arabic, methylcellulose, carboxymethylcellulose, sodium carboxymethylcellulose, crystalline cellulose, D-mannitol, trehalose, hydroxypropylcellulose, hydroxypropylmethylcellulose, polyvinylpyrrolidone, polyvinyl alcohol and the like can be mentioned; as the lubricant, stearic acid, fatty acid salts such as stearate and the like, talc, silicates and the like can be mentioned; as the solvent, purified water, saline and the like can be mentioned; as the disintegrant, low substituted hydroxypropylcellulose, chemically modified cellulose, starches and the like can be mentioned; as the dissolution aids, polyethylene glycol, propylene glycol, trehalose, benzyl benzoate, ethanol, sodium carbonate, sodium citrate, sodium salicylate, sodium acetate and the like can be mentioned; as the suspending agent or emulsifier, sodium lauryl sulfate, gum arabic, gelatin, lecithin, glyceryl monostearate, polyvinyl alcohol, polyvinyl pyrrolidone, celluloses such as sodium carboxymethylcellulose and the like, polysorbates, hydrogenated polyoxyethylene castor oil and the like can be mentioned; as the isotonizing agent, sodium chloride, potassium chloride, saccharide, glycerine, urea and the like can be mentioned; as the stabilizer, polyethylene glycol, sodium dextran sulfate, other amino acids and the like can be mentioned; as the soothing agent, glucose, calcium gluconate, procain hydrochloride and the like can be mentioned; as the preservative, paraoxybenzoates, chlorobutanol, benzyl alcohol, phenethyl alcohol, dehydroacetic acid, sorbic acid and the like can be mentioned; as the antioxidant, subsulfate, ascorbic acid and the like can be mentioned; as the corrigent, sweetener, flavoring and the like conventionally used in the fields of pharmaceutical agents and food can be mentioned; and as the coloring agent, artificial color conventionally used in the fields of pharmaceutical agents and food can be mentioned.

Since the active ingredient of the agent or composition of the present invention is amino acid having superior safety, in one embodiment, the agent or composition of the present invention can be provided as a food or drink. When the agent or composition of the present invention is provided as a food or drink, no particular difficulty occurs and, it can be prepared by, for example, mixing the active ingredient branched chain amino acid (preferably isoleucine or a salt thereof, leucine or a salt thereof, and valine or a salt thereof) with foods and drinks such as juice, milk, confectionery, jelly and the like. Vitamins and other supplements may also be added. The obtained food and drink can also be provided as a food with health claims.

The food with health claims is a food containing health function components that influence physiological functions or biological activities of the body, and is expected to achieve, by ingestion, a particular health object of a person who ingests same for such health object in a dietary life.

In some cases, food with health claims is sometimes referred to as functional food, health food (including nutrition supplements) and the like. The functional food refers to a food produced by utilizing the function of the biological regulation components contained in the food, and the health food refers to foods generally considered to be useful for the promotion of good health. They also include nutrition supplements containing a particular nutrition source as a main component.

The food with health claims includes a food for specified health uses and a food with nutrient function claims, and ingestion thereof as a daily nutritional food by patients affected with a cancer containing cancer stem cells and under administration of a chemotherapeutic agent, or patients with a history of having a cancer containing cancer stem cells can enhance the sensitivity of the cancer to the chemotherapeutic agent.

Such food with health claims can be provided with an indication that it is used for potentiating the antitumor activity of a chemotherapeutic agent against a cancer containing cancer stem cells.

While the content of the branched chain amino acid (preferably, isoleucine or a salt thereof, leucine or a salt thereof, and valine or a salt thereof) contained in the agent or composition of the present invention varies depending on the form of the preparation and the like, it is generally 1 - 100 wt%, preferably 10 - 100 wt%, more preferably 30 - 100 wt%, further preferably 50 - 100 wt%, relative to the whole preparation.

Preferable examples of the agent and composition of the present invention include branched chain amino acid preparation LIVACT (registered trade mark) granules (Ajinomoto Co., Inc.) (agent for oral administration) containing isoleucine, leucine and valine at a weight ratio of 1:2:1.2 (isoleucine: 0.952 g, leucine: 1.904 g, valine: 1.144 g).

As preferable agents for parenteral administration, high concentration-amino acid infusions such as AMINIC ((registered trade mark) drip intravenous injection (Ajinomoto Co., Inc.)), and MORIHEPAMIN ((registered trade mark) drip intravenous injection (Ajinomoto Co., Inc.)) can be mentioned.

While the dosage of the agent or composition of the present invention varies depending on the age, body weight, disease state of the subject patient, administration method and the like, in an embodiment containing isoleucine or a salt thereof, leucine or a salt thereof, and valine or a salt thereof, a daily dose for a normal adult human is generally isoleucine 0.5 - 30.0 g, leucine 1.0 - 60.0 g and valine 0.5 - 30.0 g. Preferably, a daily dose is isoleucine 2.0 - 10.0 g, leucine 3.0 - 20.0 g and valine 2.0 - 10.0 g, more preferably isoleucine 2.5 - 3.5 g, leucine 5.0 - 7.0 g and valine 3.0 - 4.0 g. The total amount of the three kinds of branched chain amino acids is preferably about 2.0 g - 50.0 g per day, which is administered in 1 - 6 portions, preferably 1 - 3 portions, where necessary. When the agent or composition of the present invention contains a salt of a branched chain amino acid, the dose is calculated by converting the salt of each branched chain amino acid to a free form.

When the above-mentioned dose (ingestion amount) of the branched chain amino acid, which is the active ingredient used in the present invention, is calculated, since the aforementioned calculation range has been determined as the active ingredient of a medicament used for the treatment, prophylaxis and the like of the object disease in the present invention, a branched chain amino acid ingested or administered for an object different from the above, for example, a need for normal dietary life, or treatment of a different disease, does not need to be included in the aforementioned calculation. For example, it is not necessary to subtract the amount of branched chain amino acid ingested per day in normal dietary life from the daily dose of the aforementioned active ingredient in the present invention.

In an embodiment where the agent of the present invention contains two or three kinds of branched chain amino acids selected from isoleucine, leucine and valine, or a salt thereof, isoleucine, leucine and valine or salts thereof, which are the active ingredients of the present invention, may be each formulated singly into a preparation, or any combination thereof may be contained in a preparation. Alternatively, all of them may be contained in one preparation. For administration of separately-formulated preparations, the administration route and administration dosage form thereof may be the same or different. In addition, the timing of administration thereof may be simultaneous or different. They are appropriately determined in consideration of the kind and effect of the pharmaceutical agent to be used concurrently. That is, the agent of the present invention may be a preparation simultaneously containing plural branched chain amino acids or a salt thereof, or a concomitant drug using separately-formulated preparations in combination. All of these forms are encompassed in the agent. Particularly, an embodiment wherein all branched chain amino acids or a salt thereof are contained in a single preparation (e.g., an embodiment of a single preparation containing isoleucine or a salt thereof, leucine or a salt thereof, and valine or a salt thereof) is preferable since it can be administered conveniently.

In the present invention, the "weight ratio" means the weight ratio of each amino acid component in the agent and composition of the present invention. For example, when each ingredient of isoleucine, leucine and valine is contained in a single preparation, the ratio is that of individual contents. When each or any combination of the active ingredients is contained in plural preparations, it is the ratio of the weight of each active ingredient contained in each preparation.

In the present invention, moreover, the actual dose ratio is that of a single dose or daily dose of each active ingredient for each administration subject (i.e., patient). For example, when respective active ingredients of isoleucine, leucine and valine are contained in a single preparation and administered to an administration subject, the weight ratio corresponds to a dose ratio. When respective active ingredients are contained in plural preparations singly or in any combination and administered, a ratio of the total amount of respective active ingredients in respective preparations to be administered singly or daily corresponds to a weight ratio.

Isoleucine, leucine and valine have already been widely used in the fields of medicament and food and the safety thereof has been established. For example, the acute toxicity (LD50) of the agent and composition of the present invention, which contain these branched chain amino acids at a ratio of 1:2:1.2, is not less than 10 g/Kg by oral administration to mouse.

As mentioned above, at least one kind of branched chain amino acid selected from isoleucine, leucine and valine, or a salt thereof (preferably a combination comprising isoleucine or a salt thereof, leucine or a salt thereof, and valine or a salt thereof) promotes differentiation of cancer stem cells into cancer non-stem cells, and potentiates the antitumor activity of a chemotherapeutic agent against a cancer containing cancer stem cells by this effect. Therefore, in one embodiment, the agent or composition of the present invention is administered to a mammal (e.g., human) affected with a cancer containing cancer stem cells. At least one kind of branched chain amino acid selected from isoleucine, leucine and valine, or a salt thereof (preferably a combination comprising isoleucine or a salt thereof, leucine or a salt thereof, and valine or a salt thereof) acting on the cancer stem cells contained in the cancer in the mammal promotes differentiation of the cancer stem cells into cancer non-stem cells and enhances the antitumor activity of a chemotherapeutic agent against the cancer.

In a further embodiment, at least one kind of branched chain amino acid selected from isoleucine, leucine and valine, or a salt thereof (preferably a combination comprising isoleucine or a salt thereof, leucine or a salt thereof, and valine or a salt thereof) promotes differentiation of cancer stem cells into cancer non-stem cells, suppresses expression of an adhesion molecule involved in the metastasis of cancer such as CD44 and the like, and suppresses the metastatic ability of the cancer containing the cancer stem cells by this effect. Therefore, in one embodiment, the agent or composition of the present invention is administered to a mammal (e.g., human) affected with a cancer containing cancer stem cells. At least one kind of branched chain amino acid selected from isoleucine, leucine and valine, or a salt thereof (preferably a combination comprising isoleucine or a salt thereof, leucine or a salt thereof, and valine or a salt thereof) acting on the cancer stem cells contained in the cancer in the mammal promotes differentiation of the cancer stem cells into cancer non-stem cells and suppresses metastasis of the cancer even without requiring a combined use of a chemotherapeutic agent.

In another embodiment, the agent or composition of the present invention is administered to a mammal (e.g., human) with a history of having a cancer containing cancer stem cells. Cancer stem cells are generally slow in the growth speed and resistant to chemotherapeutic agents. Therefore, once a cancer containing cancer stem cells is developed, even when the patient shows apparent remission of cancer and the cancer symptoms disappear as a result of treatments with chemotherapeutic agents and the like, cancer stem cells are considered to still remain in the body and cause metastasis and recurrence of the cancer. Recurrence of cancer means that cancer cells grow after the symptoms of cancer show complete or partial remission by treatments, and the symptoms of cancer appear again or are aggravated. Therefore, by administering the agent or composition of the present invention to a mammal (e.g., human) with a history of having a cancer containing cancer stem cells, differentiation of cancer stem cells possibly remaining in the body of the mammal into cancer non-stem cells is promoted and sensitivity to chemotherapeutic agents is potentiated.

In a preferable embodiment, therefore, the agent or composition of the present invention is administered to a mammal in combination with a chemotherapeutic agent. By administering the agent or composition of the present invention in combination with a chemotherapeutic agent to a mammal affected with a cancer containing cancer stem cells, the cancer can be efficiently treated. Moreover, by administering the agent or composition of the present invention in combination with a chemotherapeutic agent to a mammal with a history of having a cancer containing cancer stem cells, metastasis and recurrence of the cancer can be efficiently prevented.

Therefore, the present invention also provides an agent (or composition) for treating a cancer containing cancer stem cells and/or an agent (or composition) for preventing metastasis or recurrence of a cancer containing cancer stem cells, which comprise(s) at least one kind of branched chain amino acid selected from isoleucine, leucine and valine, or a salt thereof (preferably isoleucine or a salt thereof, leucine or a salt thereof, and valine or a salt thereof) (hereinafter sometimes to be simply referred to as branched chain amino acid) and a chemotherapeutic agent in combination (hereinafter these preparations are to be also referred to as the prophylactic or therapeutic agent of the present invention).

Unless otherwise specified, the definition of each term relating to the prophylactic or therapeutic agent of the present invention is the same as the definition of each term relating to the aforementioned agent or composition of the present invention.

The cancer to which the prophylactic or therapeutic agent of the present invention is applicable is the same as the cancer to which the above-mentioned agent or composition of the present invention is applicable. In one embodiment, the cancer containing cancer stem cells to which the agent of the present invention is applicable is resistant to a chemotherapeutic agent.

Among the cancers, hepatic cancer, colorectal cancer, renal cancer, melanoma, pancreatic cancer, thyroid cancer, gastric cancer, lung cancer, breast cancer and non-small cell lung cancer are generally known to have low sensitivity to chemotherapeutic agents, and respond poorly to chemotherapeutic agents. Therefore, when the prophylactic or therapeutic agent of the present invention is applied to these cancers (preferably gastric cancer, hepatic cancer, breast cancer and colorectal cancer), the branched chain amino acid (preferably, isoleucine or a salt thereof, leucine or a salt thereof, and valine or a salt thereof) contained in the prophylactic or therapeutic agent of the present invention promotes differentiation of cancer stem cells contained in the cancer to achieve high sensitivity of these cancers to chemotherapeutic agents. Then, the chemotherapeutic agent contained in the prophylactic or therapeutic agent of the present invention acts thereon, as a result of which the cancer stem cells are expected to be killed effectively.

In one embodiment, the prophylactic or therapeutic agent of the present invention contains antimetabolite (preferably pyrimidine antimetabolite, more preferably 5-fluorouracil or tegafur) as a chemotherapeutic agent, and is for preventing or treating hepatic cancer containing hepatic cancer stem cells.

In one embodiment, the prophylactic or therapeutic agent of the present invention contains antimetabolite (preferably pyrimidine antimetabolite, more preferably 5-fluorouracil or tegafur) as a chemotherapeutic agent, and is for preventing or treating colorectal cancer containing colorectal cancer stem cells.

In a further embodiment, the prophylactic or therapeutic agent of the present invention contains antimetabolite (preferably pyrimidine antimetabolite, more preferably 5-fluorouracil or tegafur) as a chemotherapeutic agent, and is for preventing or treating gastric cancer containing gastric cancer stem cells.

In one embodiment, the prophylactic or therapeutic agent of the present invention contains antimetabolite (preferably pyrimidine antimetabolite, more preferably 5-fluorouracil or tegafur) as a chemotherapeutic agent, and is for preventing or treating gastric cancer containing gastric cancer stem cells, colon cancer containing colon cancer stem cells, cervical cancer containing cervical cancer stem cells, endometrial cancer containing endometrial cancer stem cells, gastrointestinal cancer containing gastrointestinal cancer stem cells, pancreatic cancer containing pancreatic cancer stem cells, rectal cancer containing rectal cancer stem cells, breast cancer containing breast cancer stem cells, or ovarian cancer containing ovarian cancer stem cells.

For administration of a combination of the branched chain amino acid and a chemotherapeutic agent, the timing of the administration of the branched chain amino acid and the chemotherapeutic agent is not limited, and the branched chain amino acid and the chemotherapeutic agent may be administered to the subject simultaneously, or may be administered in a staggered manner. The doses of the branched chain amino acid and the chemotherapeutic agent are not particularly limited as long as the object effect (effect of treating cancer containing cancer stem cells, effect of preventing metastasis or recurrence of cancer containing cancer stem cells) can be achieved, and can be appropriately selected depending on the subject of administration, administration route, symptom, combination and the like. The branched chain amino acid is preferably administered in an amount that promotes differentiation of cancer stem cells into cancer non-stem cells, and the chemotherapeutic agent is administered in an amount that kills said cancer non-stem cells.

The administration mode of the branched chain amino acid and the chemotherapeutic agent is not particularly limited, and it is sufficient as long as the branched chain amino acid and the chemotherapeutic agent are combined in administration. Examples of such administration mode include (1) administration of a single preparation obtained by simultaneously processing the branched chain amino acid and the chemotherapeutic agent, (2) simultaneous administration of two kinds of preparations of the branched chain amino acid and the chemotherapeutic agent, which have been separately produced, by the same administration route, (3) administration of two kinds of preparations of the branched chain amino acid and the chemotherapeutic agent, which have been separately produced, by the same administration route in a staggered manner, (4) simultaneous administration of two kinds of preparations of the branched chain amino acid and the chemotherapeutic agent, which have been separately produced, by different administration routes, (5) administration of two kinds of preparations of the branched chain amino acid and the chemotherapeutic agent, which have been separately produced, by different administration routes in a staggered manner (for example, administration in the order of the branched chain amino acid and the chemotherapeutic agent, or in the reverse order) and the like.

The mode of administration and dosage form of the prophylactic or therapeutic agent of the present invention may be any of oral administration and parenteral administration, and as an agent for oral administration, solids such as powder, granule, capsule, tablet, chewable agent and the like and liquids such as solution, syrup and the like can be mentioned, and as an agent for parenteral administration, injection, transfusion, transnasal or transpulmonary spray and the like can be mentioned. The agent or composition of the present invention can be formulated as a medicament in these dosage forms by a general method.

When necessary for formulation, moreover, the prophylactic or therapeutic agent of the present invention is produced by mixing with appropriate pharmacologically acceptable carriers, such as excipient, binder, lubricant, solvent, disintegrant, dissolution aids, suspending agent, emulsifier, isotonizing agent, stabilizer, soothing agent, preservative, antioxidant, corrigent, coloring agent and the like.

When the branched chain amino acid and a chemotherapeutic agent are separately formulated, the content of the branched chain amino acid in a preparation containing the branched chain amino acid is the same as in the aforementioned agent or composition of the present invention.

When the branched chain amino acid and a chemotherapeutic agent are separately formulated, the content of the chemotherapeutic agent in a preparation containing the chemotherapeutic agent is not particularly limited as long as the combined use with the branched chain amino acid can treat a cancer containing cancer stem cells, or can prevent metastasis or recurrence of a cancer containing cancer stem cells, and varies depending on the preparation form and the kind of the chemotherapeutic agent. It is generally about 0.1 - 99.9 wt%, preferably about 1 - 99 wt%, more preferably about 10 - 90 wt%, of the whole preparation.

When a branched chain amino acid and a chemotherapeutic agent are simultaneously formulated and used as a single preparation, the contents may also follow the above-mentioned contents. In this case, the mixing ratio of the branched chain amino acid and the chemotherapeutic agent can be appropriately selected according to the administration subject, administration route, symptom, kind of chemotherapeutic agent and the like.

The dose of the branched chain amino acid is the same as that of the branched chain amino acid in the aforementioned agent or composition of the present invention. In an embodiment containing isoleucine or a salt thereof, leucine or a salt thereof, and valine or a salt thereof, a daily dose for a normal adult human is generally isoleucine 0.5 - 30.0 g, leucine 1.0 - 60.0 g and valine 0.5 - 30.0 g. Preferably, a daily dose is isoleucine 2.0 - 10.0 g, leucine 3.0 - 20.0 g and valine 2.0 - 10.0 g, more preferably isoleucine 2.5 - 3.5 g, leucine 5.0 - 7.0 g and valine 3.0 - 4.0 g. The total amount of the three kinds of branched chain amino acids is preferably about 2.0 g - 50.0 g per day, which is administered in 1 - 6 portions, preferably 1 - 3 portions, where necessary.

The dose of the chemotherapeutic agent is not particularly limited as long as the combined use with the branched chain amino acid can treat a cancer containing cancer stem cells, or can prevent metastasis or recurrence of a cancer containing cancer stem cells, and can be appropriately selected according to the administration subject, symptom, administration route, kind of antitumor agent and the like.

While the administration frequency of the branched chain amino acid and/or chemotherapeutic agent varies depending on the administration subject, symptom, administration route, kind of antitumor agent and the like, it is, for example, once in 1 - 7 days, preferably 1 - 3 days. While the number of administration of the branched chain amino acid and/or the chemotherapeutic agent varies depending on the administration subject, symptom, administration route, kind of antitumor agent and the like, it is generally 1 - 15 times, preferably about 2 - 10 times.

When the aforementioned branched chain amino acid and the chemotherapeutic agent are separately formulated and administered in combination, a preparation containing the branched chain amino acid may be administered after administration of a preparation containing the chemotherapeutic agent or a preparation containing the chemotherapeutic agent may be administered after administration of a preparation containing the branched chain amino acid, though they may be administered simultaneously. When administered in a staggered manner, the interval varies depending on the effective ingredient to be administered, drug form and administration method, and for example, when a preparation containing the branched chain amino acid is administered first, a method in which a preparation containing the chemotherapeutic agent is administered within a time range of from 1 min to 14 days after administration of a preparation containing the branched chain amino acid can be mentioned. When a preparation containing the chemotherapeutic agent is administered first, a method in which a preparation containing the branched chain amino acid is administered within a time range of from 1 min to 14 days after administration of a preparation containing the chemotherapeutic agent can be mentioned.

As mentioned above, since cancer stem cells are slow in the growth speed, they are resistant to chemotherapeutic agents. In contrast, since differentiated cancer cells (cancer non-stem cells) generally show a high growth speed, they are highly sensitive to chemotherapeutic agents. Therefore, by applying at least one kind of branched chain amino acids selected from isoleucine, leucine and valine, or a salt thereof (preferably isoleucine or a salt thereof, leucine or a salt thereof, and valine or a salt thereof) to the cancer stem cells to promote differentiation of cancer stem cells into cancer non-stem cells, the sensitivity of the cancer containing cancer stem cells to chemotherapeutic agents is enhanced and, as a result, the cancer containing cancer stem cells can be killed highly efficiently.

Based on such theory, a chemotherapeutic agent is preferably administered simultaneously with the administration of the aforementioned branched chain amino acid (preferably, isoleucine or a salt thereof, leucine or a salt thereof, and valine or a salt thereof) or a given time after the administration of the aforementioned branched chain amino acid (preferably, isoleucine or a salt thereof, leucine or a salt thereof, and valine or a salt thereof). That is, an embodiment of the administration of the therapeutic or prophylactic agent of the present invention preferably includes a step of simultaneously administering the aforementioned branched chain amino acid (preferably, isoleucine or a salt thereof, leucine or a salt thereof, and valine or a salt thereof) and a chemotherapeutic agent, or a step of administering the aforementioned branched chain amino acid (preferably, isoleucine or a salt thereof, leucine or a salt thereof, and valine or a salt thereof) and thereafter administering a chemotherapeutic agent.

Therefore, the administration protocol of the prophylactic or therapeutic agent of the present invention preferably includes the steps of
(1) administering once or plural times the aforementioned branched chain amino acid (preferably, isoleucine or a salt thereof, leucine or a salt thereof, and valine or a salt thereof) simultaneously with a chemotherapeutic agent,
(2) administering once or plural times the aforementioned branched chain amino acid (preferably, isoleucine or a salt thereof, leucine or a salt thereof, and valine or a salt thereof) as a first stage, and administering once or plural times a chemotherapeutic agent as a second stage,
(3) administering once or plural times the aforementioned branched chain amino acid (preferably, isoleucine or a salt thereof, leucine or a salt thereof, and valine or a salt thereof) as a first stage, and administering once or plural times the aforementioned branched chain amino acid (preferably, isoleucine or a salt thereof, leucine or a salt thereof, and valine or a salt thereof) simultaneously with a chemotherapeutic agent as a second stage,
(4) repeating step (2) or (3) plural times,
and the like.

A specific embodiment of (1) is, for example, prophylaxis or treatment of hepatic cancer containing hepatic cancer stem cells, wherein a transfusion containing the aforementioned branched chain amino acid (preferably, isoleucine or a salt thereof, leucine or a salt thereof, and valine or a salt thereof) is intraarterially injected together with a chemotherapeutic agent when a intra-hepatic artery injection chemotherapy is performed. Furthermore, interferon may be intraarterially injected together with the above-mentioned transfusion and chemotherapeutic agent.

In (2) - (4), while the interval between the final administration in the first stage and the final administration in the second stage varies depending on the administration subject, symptom, administration route, kind of chemotherapeutic agent and the like, it is generally within 1 min - 14 days.

In (2) - (4), whether the cancer stem cells were differentiated into cancer non-stem cells may be confirmed after administration of the aforementioned branched chain amino acid (preferably, isoleucine or a salt thereof, leucine or a salt thereof, and valine or a salt thereof) in the first stage. Such confirmation can be performed by, for example, measurement of, as mentioned above, the expression of a marker of the cancer stem cells by an immunological method using an antibody that specifically recognizes said marker protein. When expression of the markers of cancer stem cells in cancer decreases after administration of the aforementioned branched chain amino acid as compared to that before the administration, it can be considered that differentiation of cancer stem cells into cancer non-stem cells was induced by the administration. After confirmation of the induction of differentiation of the cancer stem cells into cancer non-stem cells, administration of a chemotherapeutic agent in the second stage can be performed.

In a further embodiment, by administering the agent or composition of the present invention to a mammal (e.g., human) with a history of having a cancer containing cancer stem cells, differentiation of cancer stem cells possibly remaining in the body of the mammal into cancer non-stem cells is promoted and expression of an adhesion molecule involved in the metastasis of cancer, such as CD44 and the like, is suppressed, and suppresses the metastatic ability of the cancer containing the cancer stem cells by this effect. Therefore, by administering the agent or composition of the present invention to a mammal with a history of having a cancer containing cancer stem cells, metastasis and recurrence of the cancer can be efficiently prevented, without requiring a combined use of a chemotherapeutic agent.

### Examples

The present invention is explained in more detail in the following by referring to Examples. The following Examples specifically explain one embodiment of the present invention, and do not limit the present invention.

### Example 1: Effect of BCAA on the expression of stem cell markers and differentiation markers in human hepatic cancer cell line HAK4

Human hepatic cancer cell line HAK4 cells were plated in a plate at a concentration of 12000 cells/well, and cultured in 2 mM BCAA or LC 2.5% medium for 72 hr. BCAA is a mixture of isoleucine, leucine and valine (weight ratio: isoleucine:leucine:valine=1:2:1.2). The concentration of BCAA, "2 mM", is a total concentration of all amino acids of isoleucine, leucine and valine.

The composition of the LC 2.5% medium is shown in the following Table. Note that "2.5%" of LC 2.5% is the content of bovine serum.

**Table 1**

| | composition (nmol/ml) |
|---|---|
| Glycine | 280 |
| L-Alanine | 307 |
| L-Serine | 151 |
| L-Threonine | 138 |
| L-Cystine 2HCl | 67 |
| L-Methionine | 75 |
| L-Glutamine | 689 |
| L-Asparagine | 64 |
| L-Glutamic Acid | 53 |
| L-Aspartic Acid | 4 |
| L-Valine | 175 |
| L-Leucine | 100 |
| L-Isoleucine | 53 |
| L-Phenylalanine | 99 |
| L-Tyrosine | 133 |
| L-Tryptophan | 45 |
| L-Lysine-HCl | 184 |
| L-Arginine-HCl | 92 |
| L-Histidine HCl-H₂O | 85 |
| L-Proline | 176 |
| Fischer's ratio | 1.42 |

The Fischer's ratio means Valine + Leucine + Isoleucine/Tyrosine + Phenylalanine.

After 72 hr culture, the cells were recovered, lysed by adding 1 ml of ISOGEN (NIPPON GENE) to the cell pellet, and stood for 5 min at room temperature. To the obtained lysate was added 0.2 ml of chloroform, and the mixture was vigorously shaken for 15 seconds, stood at room temperature for 2 - 3 min, and centrifuged at 12000×g, 4°C for 15 min. The organic phase and the intermediate phase were removed, to the obtained aqueous phase was added 0.5 ml of isopropanol, and the mixture was stood for 5 - 10 min at room temperature, and centrifuged at 12000×g, 4°C for 10 min. The supernatant was removed, at least 1 ml of 75(v/v)% ethanol was added to the precipitate, and the mixture was vortexed and centrifuged at 7500xg, 4°C for 5 min. The supernatant was removed, the precipitate was dried for a short time, and dissolved in distilled water to give a total RNA solution.

By RT-PCR using RT-PCR 7500, mRNA expression of EpCAM (Epithelial cell adhesion molecule; marker of hepatic cancer stem cells), AFP (α-feto protein; marker of hepatic cancer and marker of hepatic cancer stem cells) and CYP3A4 (cytochrome P450 3A4; differentiation marker) was quantified. The primers used for RT-PCR are shown in the following Table.

**Table 2**

| | | |
|---|---|---|
| EpCAM F(human) | GCTGGTGTGTGAACACTGCT | (sequence No. 1) |
| EpCAM R(human) | ACGCGTTGTGATCTCCTTCT | (sequence No. 2) |
| AFP F(human) | AAATGCGTTTCTCGTTGCTT | (sequence No. 3) |
| AFP R(human) | GCCACAGGCCAATAGTTTGT | (sequence No. 4) |
| CYP3A4 F(human) | AAGGTCGCCTCAAAGAGACA | (sequence No. 5) |
| CYP3A4 R(human) | TGCACTTTCTGCTGGACATC | (sequence No. 6) |

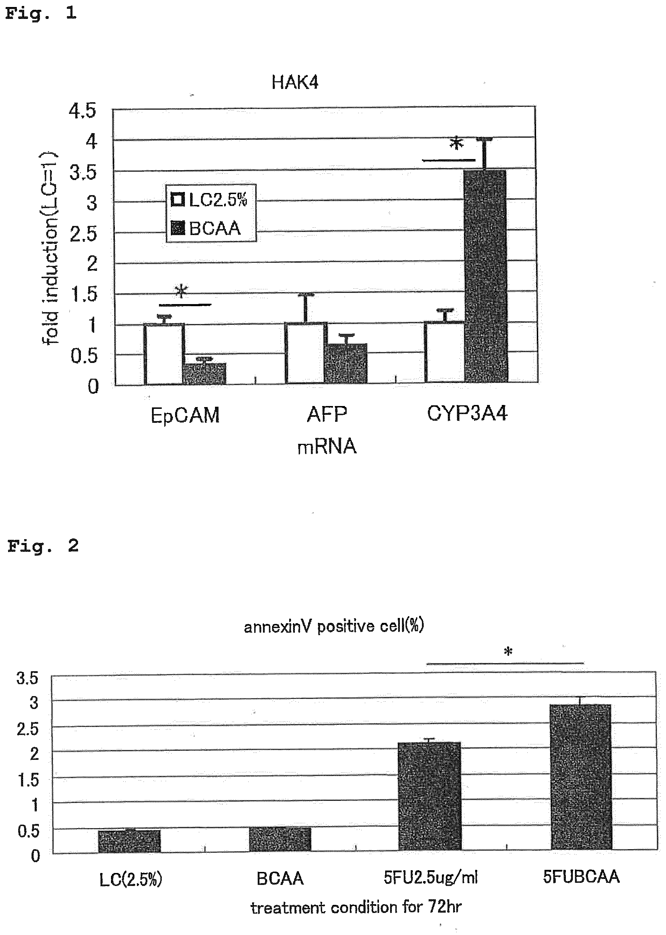

The results are shown in Fig. 1. BCAA caused a decrease in the expression of EpCAM and AFP, which are markers of hepatic cancer stem cells, and an increase in the expression of CYP3A4, which is a differentiation marker, in HAK4 cells.

### Example 2: Effect of combined use of 5FU (5-fluorouracil) and BCAA on human hepatic cancer cell line HAK4 (in vitro test)

Human hepatic cancer cell line HAK4 cells were plated at a concentration of 3000 cells/well in a plate, and cultured under the following conditions for 72 hr:
- LC 2.5% medium
- BCAA (2 mM)
- 5FU (2.5 µg/ml)
- BCAA (2 mM)+5FU (2.5 µg/ml)

After 72 hr, using ApoAlert Annexin V-FITC Apoptosis kit (Clontech), the cells were stained with annexin V and fixed, and apoptotic cells (%) were detected by Array Scan (Thermo Fisher).

To be specific, the cells in the 96 well plate were rinsed with 1X Binding Buffer, 40 µl of 1X Binding Buffer containing annexin V (1 µl) was added to each well, and the cells were incubated in the dark at room temperature for 5 - 15 min. The cells were washed, and fixed with 2% formaldehyde. 100 µl of PBS containing Hoechst33258 solution was added, and the cells were incubated in the dark for 5 min, and apoptotic cells (%) were detected by Array Scan.

The results are shown in Fig. 2. A treatment with BCAA alone did not cause a significant change in the number of apoptotic cells. A treatment with 5FU alone increased apoptotic cells and, when BCAA was use in combination, apoptosis induction by 5FU was enhanced. The above results suggest that BCAA potentiates the antitumor effect of 5FU.

### Example 3: Effect of BCAA on the expression of stem cell markers and differentiation markers in human hepatic cancer cell line HAK1B

In the same manner as in Examples 1, human hepatic cancer cell line HAK1B cells were plated in a plate at a concentration of 4000 cells/well, and cultured in 2 mM BCAA or LC 2.5% medium for 72 hr. After 72 hr culture, the cells were recovered, and the total RNA was purified by ISOGEN(NIPPON GENE). mRNA expression of EpCAM, AFP and CYP3A4 was quantified by RT-PCR.

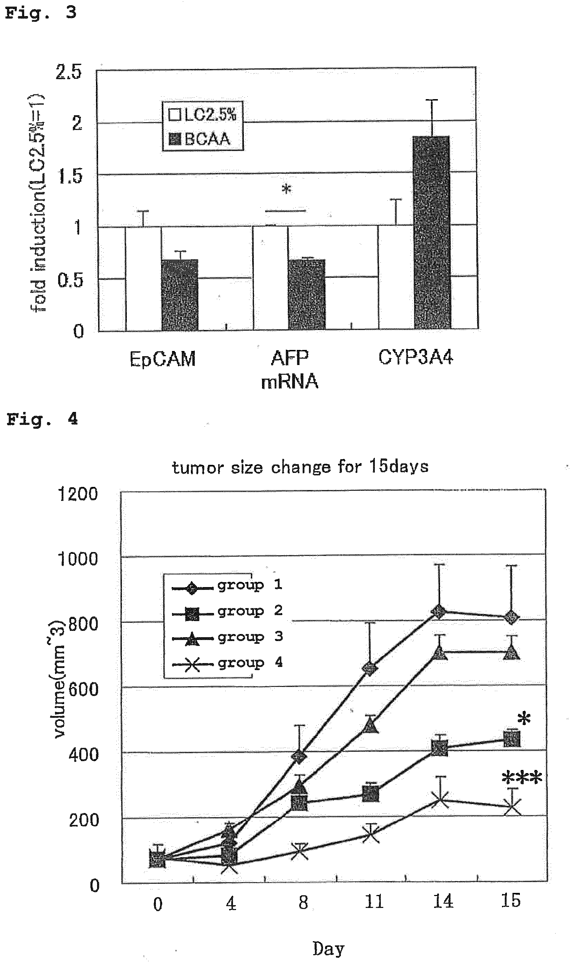

The results are shown in Fig. 3. BCAA caused a decrease in the expression of EpCAM and AFP, which are markers of hepatic cancer stem cells, and an increase in the expression of CYP3A4, which is a differentiation marker, also in HAK1B cells, as in HAK4 cells. The above results suggest that BCAA induces differentiation of cancer stem cells into cancer non-stem cells in hepatic cancer, and decreases cancer stem cells.

### Example 4: Effect of combined use of 5FU (5-fluorouracil) and BCAA on human hepatic cancer cell line HAK1B (in vivo test)

HAK1B cells were subcutaneously transplanted to 7-week-old female BALB/c nude mice (purchased from Japan Charles River) at 7,000,000 cells/mouse. After 1 week, the mice were grouped based on the tumor area. 5FU 250 µg/tumor (vehicle 10% DMSO/DW) was administered into the tumor (see Cancer Research, 70(11), 4687-4697, 2010), as well as a 3(w/w)% BCAA mixed feed or a 3(w/w)% casein mixed feed was given for 2 weeks. That is, treatments of 4 groups are as described below:
group 1: vehicle + casein mixed feed (control group)
group 2: 5FU + casein mixed feed
group 3: vehicle + BCAA mixed feed
group 4: 5FU + BCAA mixed feed

Using a vernier caliper, the tumor volume (longest diameter x shortest diameter x height (mm³)) was measured from the outer side of the tumor to monitor time-course changes in the tumor volume. On day 43, autopsy was performed, and the weight and volume of the tumor were measured.

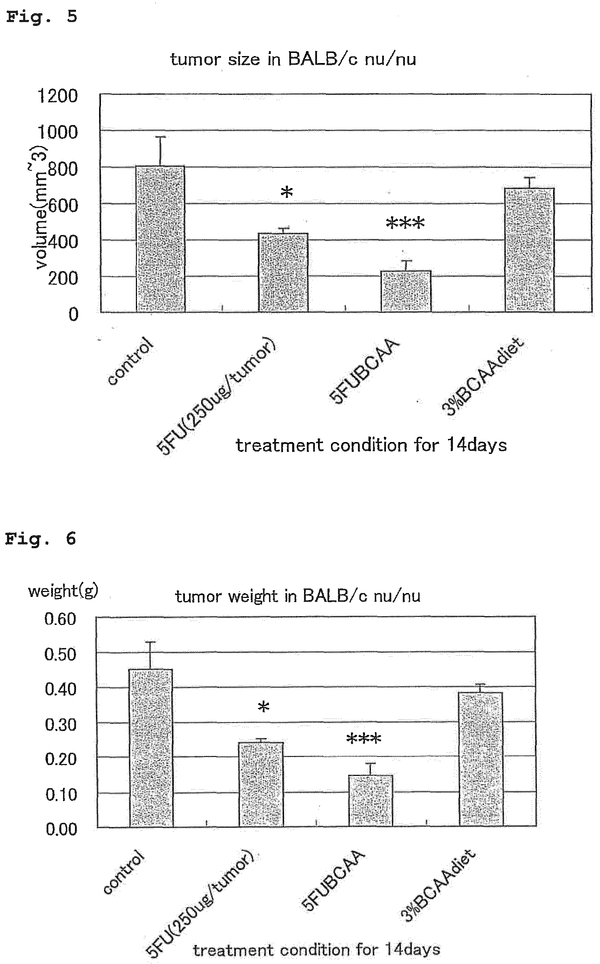

The results are shown in Figs. 4 - 6. BCAA alone treatment group (group 3) did not show a significant change in the tumor weight and tumor volume, as compared to the control group (group 1). The tumor weight and tumor volume significantly decreased by 5FU alone treatment (group 2), and when BCAA was used in combination, tumor weight and tumor volume-decreasing effect of 5FU was enhanced. The above results suggest that BCAA potentiates the antitumor effect of 5FU also in vivo.

### Example 5: Effect of BCAA on the expression of stem cell marker in human colorectal cancer cell line HCT116

HCT116 cells (human colorectal cancer cell line) were plated in a plate at a concentration of 4000 cells/well, and cultured in 2 mM BCAA or LC 2.5% medium for 72 hr. After 72 hr culture, the cells were recovered and incubated together with an anti-CD44 antibody (primary antibody) for 1 hr at room temperature, and further with a Cy5-coupled secondary antibody for 1 hr at room temperature. The cells were fixed with 4% para-formaldehyde, stained with Hoechst 33258 solution (incubated for 5 min at room temperature), the staining solution was substituted by PBS, and the ratio of CD44 positive cells was analyzed by Array Scan. CD44 is a colorectal cancer stem cell marker.

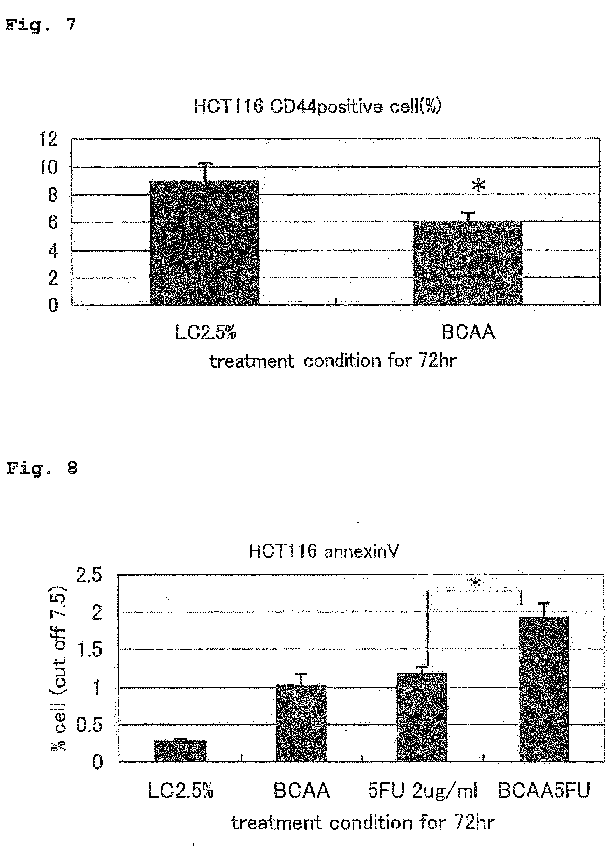

The results are shown in Fig. 7. BCAA caused a significant decrease in the expression of CD44, which is a colorectal cancer stem cell marker, in HCT116 cells. The above results suggest that BCAA induces differentiation of not only hepatic cancer stem cells but also colorectal cancer stem cells into non-stem cells.

### Example 6: Effect of combined use of 5FU and BCAA on human colorectal cancer cell line HCT116 (in vitro test)

HCT116 cells (human colorectal cancer cell line) were plated at a concentration of 4000 cells/well in a plate, and cultured under the following conditions for 72 hr:
- LC 2.5% medium
- BCAA (2 mM)
- 5FU (2 µg/ml)
- BCAA (2 mM)+5FU (2 µg/ml)

After 72 hr, using ApoAlert Annexin V-FITC Apoptosis kit (Clontech), the cells were stained with annexin V and fixed, and apoptotic cells (%) were detected by Array Scan (Thermo Fisher).

The results are shown in Fig. 8. A treatment with 5FU alone increased apoptotic cells and, when BCAA was use in combination, apoptosis induction by 5FU was enhanced. The above results suggest that BCAA potentiates the antitumor effect of 5FU not only in hepatic cancer but also colorectal cancer.

### Example 7: Effect of BCAA on metastasis of human colorectal cancer cell line HCT116 (in vivo test)

Human colorectal cancer cells HCT116 were transplanted to BALB/c nude mice (female, 6-week-old, 5 per group) at 1x10⁵ cells/mouse. HCT116 cells were treated in advance for 7 days with 4 mM BCAA. In the control group, HCT116 cells were not treated with 4 mM BCAA. For cell transplantation, the muscle layer of the mouse was incised to expose the spleen, the both ends of the blood vessels of the spleen was gently tied with a thread, HCT116 cells were transferred slowly into the spleen with an injection needle (100 µl), the thread on the both ends was tied for hemostasis, the spleen was isolated, the muscle layer was sutured and the skin was stapled. The mice were normally reared for 3 weeks, euthanized and the number of metastatic cancer on the liver surface was counted.

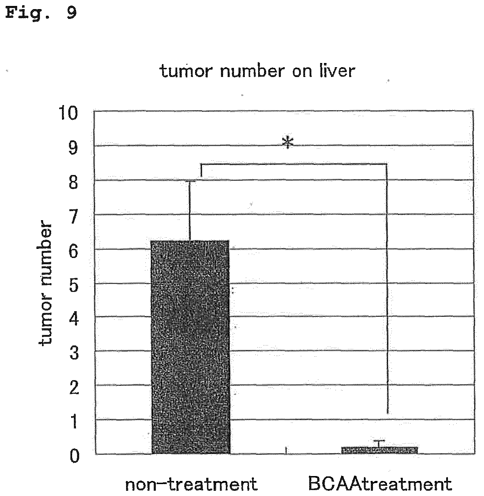

As a result, in the control group, formation of metastatic cancer on the liver surface was found in all 5 mice, and formation of metastatic cancer was found in only one mouse in the BCAA treatment group. The number of metastatic cancer per mouse was also suppressed significantly by the BCAA treatment (Fig. 9). The above results suggest that BCAA decreases metastatic ability of colorectal cancer. Combined with the results of Example 5, the metastasis suppressive effect of BCAA on colorectal cancer is suggested to be attributable to a decrease in the expression of CD44.

### Example 8: Effect of combined use of 5FU and BCAA on human gastric cancer cell line MKN45 (in vitro test)

MKN45 cells (human gastric cancer cell line) were plated in a 96 well plate at a concentration of 4000 cells/100 µl/well, and cultured under the following conditions for 72 hr:
- LC medium (10% FBS)
- LC medium +BCAA (2 mM or 4 mM)
- LC medium +5FU (0.05 µg/ml, 0.1 µg/ml, 0.5 µg/ml or 1 µg/ml)
- LC medium +BCAA (2 mM or 4 mM)+5FU (0.05 µg/ml, 0.1 µg/ml, 0.5 µg/ml or 1 µg/ml)

After 72 hr, WST-8 reagent was added (10 µl/well) and, after color development for 1 hr, the absorbance of OD 450 nm was measured. The absorbance of each group was shown by a relative value provided that the absorbance of a non-stimulation group was assumed to be 100%. WST-8 is reduced by intracellular dehydrogenase to produce water-soluble formazan. By directly measuring the absorbance of formazan at 450 nm, the number of viable cells can be easily measured. The cell number and the amount of produced formazan are in a linear proportional correlation.

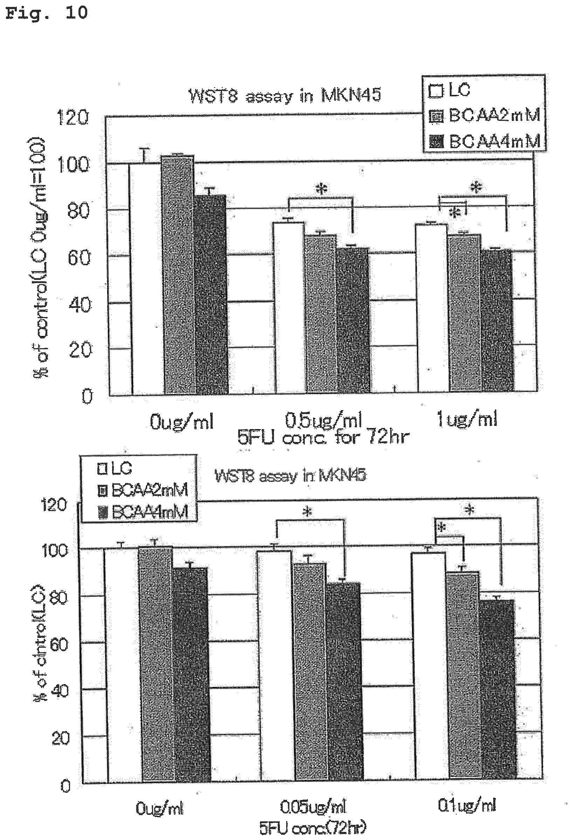

The results are shown in Fig. 10. The number of viable cells decreased by a treatment with 5FU alone and, when BCAA was used in combination, the decrease in the number of viable cells by 5FU was enhanced. The above results suggest that BCAA potentiates the antitumor effect of 5FU also in gastric cancer.

### Example 9: Effect of combined use of 5FU and BCAA on human gastric cancer cell line MKN45 (in vitro test)

MKN45 cells (human gastric cancer cell line) were plated in a 96 well plate at a concentration of 4000 cells/100 µl/well, and cultured under the following conditions for 72 hr:
- LC medium (10% FBS)
- LC medium +BCAA (2 mM or 4 mM)
- LC medium +5FU (0.05 µg/ml or 0.1 µg/ml)
- LC medium +BCAA (2 mM or 4 mM)+5FU (0.05 µg/ml or 0.1 µg/ml)

After 72 hr, the supernatant was removed, the cells were fixed with para-formaldehyde, the nuclear staining with Hoechst reagent was performed, and the ratio of apoptotic cells was analyzed using the cell cycle mode of ArrayScan.

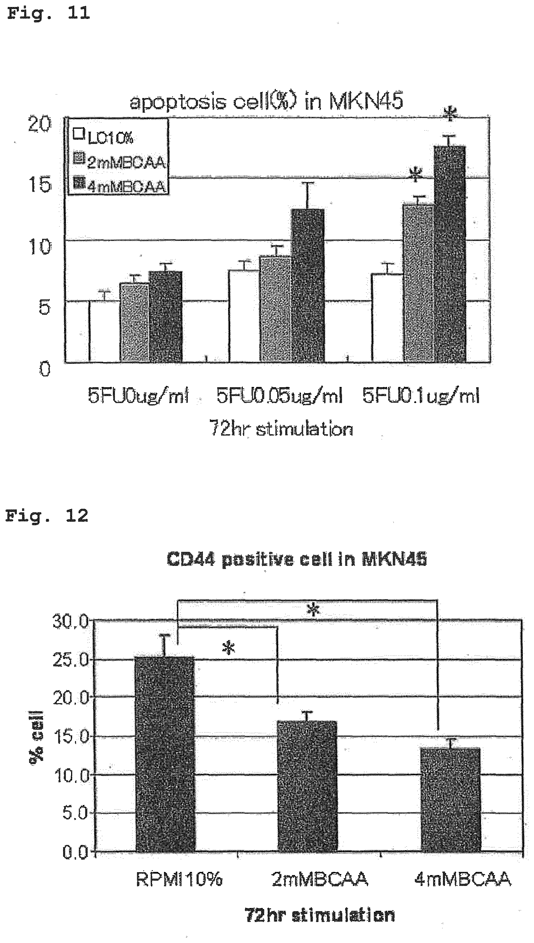

The results are shown in Fig. 11. The number of apoptotic cells significantly increased by the combined use of 5FU and BCAA. The above results suggest that the enhancement of the antitumor effect of 5FU against gastric cancer by BCAA may be attributable to the enhancement of apoptosis.

### Example 10: Effect of BCAA on the expression of stem cell marker in human gastric cancer cell line MKN45

MKN45 cells (human gastric cancer cell line) were plated in a 96 well plate at 4000 cells/100 µl/well, and cultured in RPMI1640 medium (10% FBS) or BCAA (2 mM or 4 mM) containing RPMI1640 medium (10% FBS) for 72 hr. After 72 hr, the supernatant was removed, and cells were fixed with para-formaldehyde, incubated with anti-CD44 antibody (primary antibody) for 1 hr at room temperature, and further incubated with Texas Red-conjugated secondary antibody for 1 hr. The supernatant was removed, nuclear staining with the Hoechst reagent was performed, and the ratio of CD44 positive cells was analyzed using the target activation mode of ArrayScan.

The results are shown in Fig. 12. BCAA caused a significant decrease in the expression of CD44, which is a gastric cancer stem cell marker, in MKN45 cells. The above results suggest that BCAA induces differentiation of gastric cancer stem cells into non-stem cells.

### Example 11: Effect of combined use of TS-1 and BCAA on human gastric cancer cell line MKN45 (in vivo test)

MKN45 cells were subcutaneously transplanted to 6-week-old female BALB/c nude mice at 1×10⁶ cells/mouse. After 1 week, the mice were grouped based on the tumor volume. TS-1 10 mg/kg (vehicle 0.5% CMC), TS-1 10 mg/kg+BCAA 0.75 g/kg, or BCAA 0.75 g/kg was orally administered every day for 6 weeks except Saturdays and Sundays. That is, treatments in 4 groups are as described below:
group 1: CMC (vehicle)
group 2: TS-1
group 3: TS-1+BCAA
group 4: BCAA

TS-1 is an anti-cancer agent containing tegafur, gimeracil, and oteracil potassium at the following composition. In TS-1 0.2 g

| | |
|---|---|
| tegafur | 20 mg |
| gimeracil | 5.8 mg |
| oteracil potassium | 19.6 mg |

Using a vernier caliper, the tumor volume (longest diameter x shortest diameter x height (mm³)) was measured from the outer side of the tumor to monitor time-course changes in the tumor volume. On day 43, autopsy was performed, and the weight and volume of the tumor were measured.

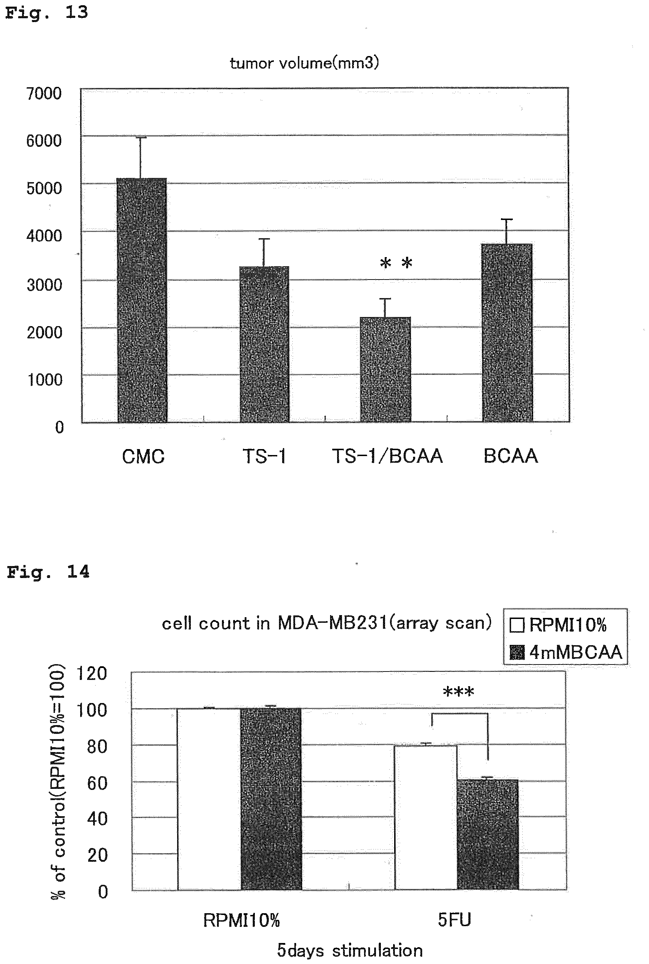

The results are shown in Fig. 13. In TS-1 or BCAA alone treatment group, a decrease tendency of tumor volume was observed as compared to CMC group, but a significant change was not observed. A combined use of TS-1 and BCAA significantly decreased the tumor volume. The above results suggest that BCAA potentiates the antitumor effect of TS-1 in vivo.

### Example 12: Effect of 5FU and BCAA on growth of human breast cancer cell line MDA-MB231 and stem cell marker expression

MDA-MB231 cells were suspended in RPMI-10% (RPMI medium containing 10% FBS), and plated in a plate at 2000 cells/well. The next day, the medium was exchanged to those shown below, and the cells were further cultured for 5 days.
group 1: RPMI-10%
group 2: RPMI-10% containing BCAA (4 mM)
group 3: RPMI-10% containing 5-FU (0.25 µg/ml)
group 4: RPMI-10% containing 5-FU (0.25 µg/ml) + BCAA (4Mm)

After 5 days, cellular nucleus was stained with Hoechst reagent, and the cell number was counted. In groups 1 and 2, cells were stained with anti-CD44 antibody, and the ratio of CD44 positive cells was analyzed by Array Scan using fluorescence microscopic quantification apparatus (Thermo Fisher).

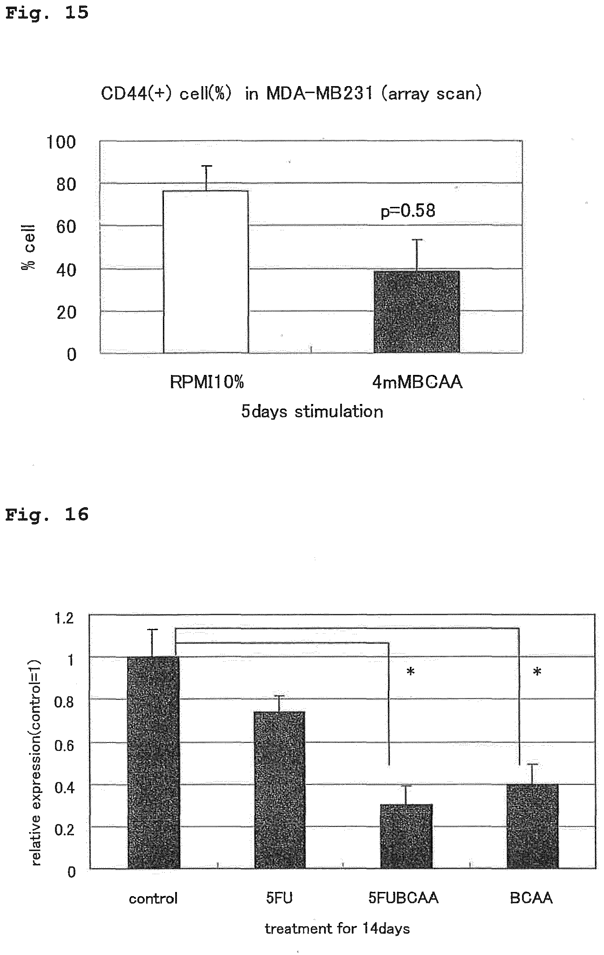

The results are shown in Figs. 14 and 15. The number of viable cells decreased by a treatment with 5FU alone and, when BCAA was used in combination, the decrease in the number of viable cells by 5FU was enhanced. The above results suggest that BCAA potentiates the antitumor effect of 5FU in breast cancer. In addition, the expression of CD44, which is a breast cancer stem cell marker, in MDA-MB231 cells tended to be decreased by BCAA. The above results suggest a possibility of BCAA inducing differentiation of breast cancer stem cells into non-stem cells.

### Example 13: Effect of BCAA on the expression of stem cell marker in human hepatic cancer cell line HAK1B (in vivo test)

HAK1B cells were subcutaneously transplanted to 7-week-old female BALB/c nude mice (purchased from Japan Charles River) at 7,000,000 cells/mouse. After 1 week, the mice were grouped based on the tumor area. 5FU 250 µg/tumor (vehicle 10% DMSO/DW) was administered into the tumor (see Cancer Research, 70(11), 4687-4697, 2010), as well as a 3(w/w)% BCAA mixed feed or a 3(w/w)% casein mixed feed was given for 2 weeks. That is, treatments in 4 groups are as described below:
group 1: vehicle + casein mixed feed (control group)
group 2: 5FU + casein mixed feed
group 3: vehicle + BCAA mixed feed
group 4: 5FU + BCAA mixed feed

On day 15, the tumor tissue was isolated, and RNA was extracted using TriPure Isolation Reagent (Roche). cDNA was synthesized using High Capacity cDNA RT kit (Applied Biosystems). The mRNA expression of EpCAM (Epithelial cell adhesion molecule; marker of hepatic cancer stem cells) was quantified by RT-PCR. The primers used for RT-PCR were the same as those in Example 1.

The results are shown in Fig. 16. BCAA decreased EpCAM, which is a marker of hepatic cancer stem cells, in HAK1B cells. The above results suggest that BCAA induces differentiation of cancer stem cells in hepatic cancer into cancer non-stem cells to decrease cancer stem cells, and increases the sensitivity of the tumor to 5FU also in vivo.

### Example 14: Effect of combined use of BCAA on stem cell marker expression in human gastric cancer cell line MKN45 (in vivo test)

MKN45 cells were subcutaneously transplanted to 6-week-old female BALB/c nude mice at 1×10⁶ cells/mouse. After 1 week, the mice were grouped based on the tumor volume. TS-1 10 mg/kg (vehicle 0.5% CMC), TS-1 10 mg/kg+BCAA 0.75 g/kg, or BCAA 0.75 g/kg was orally administered every day for 6 weeks except Saturdays and Sundays. That is, treatments in 4 groups are as described below:
group 1: CMC (vehicle)
group 2: TS-1
group 3: TS-1+BCAA
group 4: BCAA

On day 43, the tumor tissue was isolated, RNA was extracted using TriPure Isolation Reagent (Roche). cDNA was synthesized using High Capacity cDNA RT kit (Applied Biosystems). The mRNA expression of CD44 (marker of hepatic cancer stem cells), nanog (marker of undifferentiated state) and ABCB5 (ABC transporter, expression in cancer stem cells has been reported) was quantified by RT-PCR. The primers used for RT-PCR were as described below.

**Table 3**

| | | |
|---|---|---|
| CD44: | 5'-AAGGTGGAGCAAACACAACC-3' | (sequence No. 7) |
| | 5'-AGCTTTTTCTTCTGCCCACA-3' | (sequence No. 8) |
| Nanog: | 5'-TTCCTTCCTCCATGGATCTG-3' | (sequence No. 9) |
| | 5'-TCTGCTGGAGGCTGAGGTAT-3' | (sequence No. 10) |
| ABCB5: | 5'-ATGTACAGTGGCTCCGTTCC-3' | (sequence No. 11) |
| | 5'-ACACGGCTGTTGTCACCATA-3' | (sequence No. 12) |
| β actin: | 5'-GATGAGATTGGCATGGCTTT-3' | (sequence No. 13) |
| | 5'-CACCTTCACCGTTCCAGTTT-3' | (sequence No. 14) |

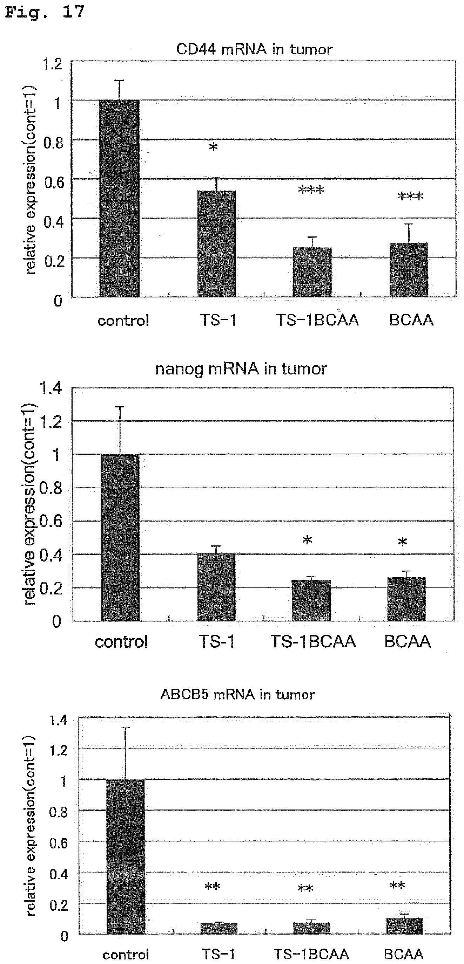

The results are shown in Fig. 17. BCAA decreased the expression of CD44, nanog and ABCB5 in MKN45 cells. The above results suggest that BCAA induces differentiation of cancer stem cells in gastric cancer into cancer non-stem cells to decrease the cancer stem cells, and increases the sensitivity of the tumor to TS-1 also in vivo.

### Industrial Applicability

Since branched chain amino acid promotes differentiation of cancer stem cells into cancer non-stem cells and enhances sensitivity to chemotherapeutic agents, cancer can be effectively treated by administering the branched chain amino acid and a chemotherapeutic agent in combination to a patient affected with a cancer containing cancer stem cells.

In addition, metastasis and recurrence of cancer can be effectively prevented by administering branched chain amino acid in combination with a chemotherapeutic agent to a patient with a history of having a cancer containing cancer stem cells.

Therefore, the present invention is useful as a medicament etc. for the prophylaxis or treatment of cancer.

This application is based on patent application Nos. 2011-32511 (filing date: February 17, 2011) and 2011-215605 (filing date: September 29, 2011) filed in Japan, the contents of which are incorporated in full herein.

## Claims

1. A composition for use in the treatment of a cancer that contains cancer stem cells, or for preventing metastasis or recurrence of such a cancer, in a mammal affected by such a cancer or with a history of having such a cancer, wherein said composition is for administration in combination with a chemotherapeutic agent, for enhancing sensitivity of said cancer to the chemotherapeutic agent, the composition comprising: isoleucine or a salt thereof, leucine or a salt thereof, and valine or a salt thereof.

2. The composition according to claim 1, wherein the chemotherapeutic agent is an antimetabolite, an alkylating agent, an antitumor antibiotic, a plant alkaloid, or a molecular target therapeutic agent.

3. The composition according to claim 2, wherein the chemotherapeutic agent is 5FU.

4. The composition according to any one of claims 1 to 3, wherein the cancer containing cancer stem cells is a chemotherapeutic agent-resistant cancer.

5. The composition according to any one of claims 1 to 4, wherein the cancer containing cancer stem cells is gastric cancer, hepatic cancer, breast cancer or colorectal cancer.

6. The composition according to any one of claims 1 to 5, wherein said isoleucine or a salt thereof, leucine or a salt thereof, and valine or a salt thereof are arranged for simultaneous or sequential administration and:
each of isoleucine or a salt thereof, leucine or a salt thereof, and valine or a salt thereof is formulated singly;
any combination of isoleucine or a salt thereof, leucine or a salt thereof, and valine or a salt thereof are formulated together in a preparation; or
all of isoleucine or a salt thereof, leucine or a salt thereof, and valine or a salt thereof are contained in a single preparation.

7. A combination for use in the treatment of a cancer that contains cancer stem cells, or for preventing metastasis or recurrence of such a cancer, in a mammal affected by such a cancer or with a history of having such a cancer, the combination including a composition according to any one of the preceding claims and a chemotherapeutic agent as set out in any of the preceding claims.

8. The combination according to claim 7, for administration to a mammal with a history of having the cancer containing cancer stem cells.

9. The combination according to claim 7 or claim 8, wherein
(1) isoleucine or a salt thereof, leucine or a salt thereof, and valine or a salt thereof are administered once or plural times with the chemotherapeutic agent,
(2) isoleucine or a salt thereof, leucine or a salt thereof, and valine or a salt thereof are administered once or plural times as a first stage, and the chemotherapeutic agent is administered once or plural times as a second stage,
(3) isoleucine or a salt thereof, leucine or a salt thereof, and valine or a salt thereof are administered once or plural times as a first stage, and isoleucine or a salt thereof, leucine or a salt thereof, and valine or a salt thereof are administered once or plural times simultaneously with the chemotherapeutic agent as a second stage, or
(4) step (2) or (3) are repeated plural times.

## Patentansprüche

1. Zusammensetzung zur Verwendung bei der Behandlung eines Krebses, der Krebsstammzellen enthält, oder zur Prävention einer Metastase oder eines Rezidivs solch eines Krebses in einem Säugetier, das von solch einem Krebs betroffen ist oder eine Vorgeschichte eines solchen Krebses hat, wobei die Zusammensetzung zur Verabreichung in Kombination mit einem Chemotherapeutikum bestimmt ist, um die Empfindlichkeit des Krebses gegenüber dem Chemotherapeutikum zu erhöhen, wobei die Zusammensetzung Folgendes umfasst: Isoleucin oder ein Salz davon, Leucin oder ein Salz davon und Valin oder ein Salz davon.

2. Zusammensetzung nach Anspruch 1, wobei das Chemotherapeutikum ein Antimetabolit, ein Alkylierungsmittel, ein Antitumorantibiotikum, ein pflanzliches Alkaloid oder ein Molekültarget-Therapeutikum ist.

3. Zusammensetzung nach Anspruch 2, wobei das Chemotherapeutikum 5FU ist.

4. Zusammensetzung nach einem der Ansprüche 1 bis 3, wobei der Krebs, der Krebsstammzellen enthält, ein Chemotherapeutikum-resistenter Krebs ist.

5. Zusammensetzung nach einem der Ansprüche 1 bis 4, wobei der Krebs, der Krebsstammzellen enthält, Magenkrebs, Leberkrebs, Brustkrebs oder Kolorektal-krebs ist.

6. Zusammensetzung nach einem der Ansprüche 1 bis 5, wobei das Isoleucin oder ein Salz davon, Leucin oder ein Salz davon und Valin oder ein Salz davon zur gleichzeitigen oder sequenziellen Verabreichung vorgesehen sind und:
das Isoleucin oder ein Salz davon, Leucin oder ein Salz davon und Valin oder ein Salz davon separat formuliert sind;
eine beliebige Kombination aus Isoleucin oder einem Salz davon, Leucin oder einem Salz davon und Valin oder einem Salz davon zusammen in einem Präparat formuliert sind; oder
Isoleucin oder ein Salz davon, Leucin oder ein Salz davon und Valin oder ein Salz davon alle in einem einzigen Präparat enthalten sind.

7. Kombination zur Verwendung bei der Behandlung eines Krebses, der Krebsstammzellen enthält, oder zur Prävention einer Metastase oder eines Rezidivs solch eines Krebses in einem Säugetier, das von solch einem Krebs betroffen ist oder eine Vorgeschichte eines solchen Krebses hat, wobei die Kombination eine Zusammensetzung nach einem der vorangegangenen Ansprüche und ein Chemotherapeutikum nach einem der vorangegangenen Ansprüche umfasst.

8. Kombination nach Anspruch 7 zur Verabreichung an ein Säugetier mit einer Vorgeschichte des Krebses, der Krebsstammzellen enthält.

9. Kombination nach Anspruch 7 oder Anspruch 8, wobei
(1) Isoleucin oder ein Salz davon, Leucin oder ein Salz davon und Valin oder ein Salz davon einmal oder mehrere Male mit dem Chemotherapeutikum verabreicht werden,
(2) Isoleucin oder ein Salz davon, Leucin oder ein Salz davon und Valin oder ein Salz davon einmal oder mehrere Male als erste Stufe und das Chemotherapeutikum einmal oder mehrere Male als zweite Stufe verabreicht werden,
(3) Isoleucin oder ein Salz davon, Leucin oder ein Salz davon und Valin oder ein Salz davon einmal oder mehrere Male als erste Stufe und Isoleucin oder ein Salz davon, Leucin oder ein Salz davon und Valin oder ein Salz davon einmal oder mehrere Male gleichzeitig mit dem Chemotherapeutikum als zweite Stufe verabreicht werden oder
(4) Schritt (2) oder (3) mehrere Male wiederholt wird.

## Revendications

1. Composition destinée à être utilisée dans le traitement d'un cancer qui contient des cellules souches cancéreuses, ou pour prévenir des métastases ou une récidive d'un tel cancer, chez un mammifère affecté par un tel cancer ou avec un historique d'un tel cancer, dans laquelle ladite composition est destinée à une administration en combinaison avec un agent chimiothérapeutique, pour améliorer la sensibilité dudit cancer à l'agent chimiothérapeutique, la composition comprenant : de l'isoleucine ou un sel de celle-ci, de la leucine ou un sel de celle-ci, et de la valine ou un sel de celle-ci.

2. Composition selon la revendication 1, dans laquelle l'agent chimiothérapeutique est un antimétabolite, un agent alkylant, un antibiotique antitumoral, un alcaloïde végétal ou un agent thérapeutique cible moléculaire.

3. Composition selon la revendication 2, dans laquelle l'agent chimiothérapeutique est du 5FU.

4. Composition selon l'une quelconque des revendications 1 à 3, dans laquelle le cancer contenant des cellules souches cancéreuses est un cancer résistant aux agents chimiothérapeutiques.

5. Composition selon l'une quelconque des revendications 1 à 4, dans laquelle le cancer contenant des cellules souches cancéreuses est un cancer de l'estomac, un cancer du foie, un cancer du sein ou un cancer colorectal.

6. Composition selon l'une quelconque des revendications 1 à 5, dans laquelle ladite isoleucine ou un sel de celle-ci, ladite leucine ou un sel de celle-ci et ladite valine ou un sel de celle-ci sont prévus (e) s pour une administration simultanée ou séquentielle, et :
chacun de l'isoleucine ou d'un sel de celle-ci, de la leucine ou d'un sel de celle-ci, et de la valine ou d'un sel de celle-ci est formulé seul ;
toute combinaison d'isoleucine ou d'un sel de celle-ci, de leucine ou d'un sel de celle-ci, et de valine ou d'un sel de celle-ci est formulée en commun dans une préparation ; ou
la totalité de l'isoleucine ou d'un sel de celle-ci, de la leucine ou d'un sel de celle-ci, et de la valine ou d'un sel de celle-ci est contenue dans une seule préparation.

7. Combinaison destinée à être utilisée dans le traitement d'un cancer qui contient des cellules souches cancéreuses, ou pour prévenir des métastases ou une récidive d'un tel cancer, chez un mammifère affecté par un tel cancer ou avec un historique d'un tel cancer, la combinaison comprenant une composition selon l'une quelconque des revendications précédentes et un agent chimiothérapeutique selon l'une quelconque des revendications précédentes.

8. Combinaison selon la revendication 7, destinée à être administrée à un mammifère ayant un historique du cancer contenant des cellules souches cancéreuses.

9. Combinaison selon la revendication 7 ou 8, dans laquelle
(1) l'isoleucine ou un sel de celle-ci, la leucine ou un sel de celle-ci, et la valine ou un sel de celle-ci sont administrés(e)s une ou plusieurs fois avec l'agent chimiothérapeutique,
(2) l'isoleucine ou un sel de celle-ci, la leucine ou un sel de celle-ci et la valine ou un sel de celle-ci sont administrés (e) s une ou plusieurs fois en tant que première étape, et l'agent chimiothérapeutique est administré une ou plusieurs fois en tant que deuxième étape,
(3) l'isoleucine ou un sel de celle-ci, la leucine ou un sel de celle-ci et la valine ou un sel de celle-ci sont administrés (e) s une ou plusieurs fois en tant que première étape, et l'isoleucine ou un sel de celle-ci, la leucine ou un sel de celle-ci et la valine ou un sel de celle-ci sont administrés(e)s une ou plusieurs fois simultanément avec l'agent chimiothérapeutique en tant que deuxième étape, ou
(4) l'étape (2) ou (3) est répétée plusieurs fois.


### SEQUENCE LISTING

<110> EA Pharma Co., Ltd. Kurume University
<120> Agent for enhancing the antitumor activity of chemotherapeutic drug
<130> 091817
<150> JP2011-032511
   <151> 2011-02-17
<150> JP2011-215605
   <151> 2011-09-29
<160> 14
<170> PatentIn version 3.4
<210> 1
   <211> 20
   <212> DNA
   <213> Artificial
<220>
   <223> forward primer for human EpCAM
<400> 1
   gctggtgtgt gaacactgct 20
<210> 2
   <211> 20
   <212> DNA
   <213> Artificial
<220>
   <223> reverse primer for human EpCAM
<400> 2
   acgcgttgtg atctccttct 20
<210> 3
   <211> 20
   <212> DNA
   <213> Artificial
<220>
   <223> forward primer for human AFP
<400> 3
   aaatgcgttt ctcgttgctt 20
<210> 4
   <211> 20
   <212> DNA
   <213> Artificial
<220>
   <223> reverse primer for human AFP
<400> 4
   gccacaggcc aatagtttgt 20
<210> 5
   <211> 20
   <212> DNA
   <213> Artificial
<220>
   <223> forward primer for human CYP 3A4
<400> 5
   aaggtcgcct caaagagaca 20
<210> 6
   <211> 20
   <212> DNA
   <213> Artificial
<220>
   <223> reverse primer for human CYP 3A4
<400> 6
   tgcactttct gctggacatc 20
<210> 7
   <211> 20
   <212> DNA
   <213> Artificial
<220>
   <223> PCR primer for human CD44
<400> 7
   aaggtggagc aaacacaacc 20
<210> 8
   <211> 20
   <212> DNA
   <213> Artificial
<220>
   <223> PCR primer for human CD44
<400> 8
   agctttttct tctgcccaca 20
<210> 9
   <211> 20
   <212> DNA
   <213> Artificial
<220>
   <223> PCR primer for human Nanog
<400> 9
   ttccttcctc catggatctg 20
<210> 10
   <211> 20
   <212> DNA
   <213> Artificial
<220>
   <223> PCR primer for human Nanog
<400> 10
   tctgctggag gctgaggtat 20
<210> 11
   <211> 20
   <212> DNA
   <213> Artificial
<220>
   <223> PCR primer for human ABCB5
<400> 11
   atgtacagtg gctccgttcc 20
<210> 12
   <211> 20
   <212> DNA
   <213> Artificial
<220>
   <223> PCR primer for human ABCB5
<400> 12
   acacggctgt tgtcaccata 20
<210> 13
   <211> 20
   <212> DNA
   <213> Artificial
<220>
   <223> PCR primer for hunan beta-actin
<400> 13
   gatgagattg gcatggcttt 20
<210> 14
   <211> 20
   <212> DNA
   <213> Artificial
<220>
   <223> PCR primer for human beta-actin
<400> 14
   caccttcacc gttccagttt 20